# EUROPEAN PATENT SPECIFICATION

(11) **EP 0 563 311 B1**
(45) Date of publication and mention of the grant of the patent: **15.03.2000**
(21) Application number: 92904089.7
(22) Date of filing: 18.12.1991
(51) Int. Cl.: C12N 15/74, A61K 39/112, A61K 39/40, C12Q 1/10, C12Q 1/68

(54) **IMPROVED VACCINES**
VERBESSERTE IMPFSTOFFE
VACCINS AMELIORES

(30) Priority: 18.12.1990 US 629602
(43) Date of publication of application: 06.10.1993
(73) Proprietor: THE GENERAL HOSPITAL CORPORATION, Charlestown, MA 02129 (US); THE PRESIDENT AND FELLOWS OF HARVARD COLLEGE, Cambridge, MA 02114 (US)
(72) Inventor: MILLER, Samuel, I., III, Brookline, MA 02146 (US); MEKALANOS, John, J., Cambridge, MA 02138 (US)
(74) Representative: Rackham, Anthony Charles
(86) International application number: US9109604
(87) International publication number: WO9211361

(56) References cited:
- WO-A-90/11687
- WO-A-92/17785
- US-A- 4 837 151
- RESEARCH IN MICROBIOLOGY, vol.141, no.7-8, 29 January 1990 pages 817 - 821 MILLER, S. ET AL. 'Salmonella vaccines with mutations in the phoP virulence region'
- J BACTERIOL 173 (1). 86-93, January 1991 PULKKINEN, W. ET AL. 'A SALMONELLA-TYPHIMURIUM VIRULENCE PROTEIN IS SIMILAR TO A YERSINIA-ENTEROCOLITICA INVASION PROTEIN AND A BACTERIOPHAGE LAMBDA OUTER MEMBRANE PROTEIN.'
- Microbial Pathogenesis, Volume 6, issued 1989, GALAN et al., "Virulence and Vaccine Potential of phoP mutants of Salmonella typhimurium", pages 433-443, see entire article.
- Infection and Immunity, Volume 58, No. 11, issued November 1990, MILLER et al., "Characterization of Defensin Ressistance Phenotypes Associated with Mutations in the phoP Virulence Regulon of Salmonella typhimurium", pages 3706-3710, see entire article.
- Proceedings of the National Academy of Sciences, Vol. 86, issued July 1989, MILLER et al., "A Two-component Regulatory System (phoP phoQ) Controls Salmonella typhimurium Virulence", pages 5054-5058, see entire article.
- Infection & Immunity, Volume 56, No. 2, issued February 1988, O'CALLAGHAN et al., "Characterization of Aromatic- and Purine-Dependent Salmonella typhimurium: Attenuation, Persistence, and Ability to Induce Protective Immunity in BALB/c Mice", pages 419-423, see entire document.
- Research Microbiology, Volume 141, issued 1990, MILLER et al., "Salmonella Vaccines with Mutations in the phoP Virulence Regulon- Salmonella typhimurium and Salmonella typhi Attenuation; Recombinant Vaccine Construction", pages 817-821, see Abstract No. 114930.
- Journal of Infectious Diseases, Vol. 158, No. 6, issued December 1988, DOUGAN et al., "Construction of Vaccine Strains...Genes", pages 1329-1335, see entire document.
- Journal of Bacteriology, Vol. 172, No. 5, issued May 1990, MILLER et al., "Constitutive Expression on the PhoP...Macrophages", pages 2485-2490, see entire document.

SEQUENCE LISTING

(1) GENERAL INFORMATION:

(i) APPLICANT: THE GENERAL HOSPITAL CORPORATION
OFFICE OF TECHNOLOGY AFFAIRS
THIRTEENTH STREET
BUILDING 149, SUITE 1101
CHARLESTOWN, MASSACHUSETTS 02129
U.S.A.

and

PRESIDENT AND FELLOWS OF HARVARD COLLEGE
17 QUINCY STREET
CAMBRIDGE, MASSACHUSETTS 02138
U.S.A.

(ii) TITLE OF INVENTION: Improved Vaccines

(iii) NUMBER OF SEQUENCES: 2

(iv) COMPUTER READABLE FORM:

(A) MEDIUM TYPE: 3.5" Diskette, 1.44 Mb storage
(B) COMPUTER: IBM PC COMPATIBLE
(C) OPERATING SYSTEM: MS-DOS (Version 5.0)
(D) SOFTWARE: CONVERTED WP51

(v) CURRENT APPLICATION DATA:

(A) APPLICATION NUMBER: EUROPEAN PATENT APPLICATION NO:
92904089.7

(2) INFORMATION FOR SEQ ID NO: 1:

(i) SEQUENCE CHARACTERISTICS:

(A) LENGTH: 2320
(B) TYPE: nucleic acid
(C) STRANDEDNESS: single
(D) TOPOLOGY: linear

(xi) SEQUENCE DESCRIPTION: SEQ ID NO: 1:

GTTAACCACT CTTAATAATA ATGGGTTTTA TAGCGAAATA CACTTTTTTA TCGCGTGTTC

AATATTTGCG TTAGTTATTA TTTTTTTGGA ATGTAAATTC TCTCTAAACA CAGGTGATAT 1

TTATGTTGGA ATTGTGGTGT TGATTCTATT CTTATAATAT AACAAGAAAT GTTGTAACTG 1

ATAGATATAT TAAAAGATTA AATCGGAGGG GGAATAAAGC GTGCTAAGCA TCATCGTGAA 2

TATGATTACA GCGCCTGCGA TGGCATATAA CCGTATTGCG GATGGAGCGT CACGTGAGGA 3

CTGTGAAGCA CAATGCGATA TGTTCTGATT ATATGGCGAG TTTGCTTAAT GACATGTTTT 3

TAGCCGAACG GTGTCAAGTT TCTTAATGTG GTTGTGAGAT TTTCTCTTTA AATATCAAAA 4

TGTTGCATGG GTGATTTGTT GTTCTATAGT GGCTAAAGAC TTTATGGTTT CTGTTAAATA 4

TATATGCGTG AGAAAAATTA GCATTCAAAT CTATAAAAGT TAGATGACAT TGTAGAACCG 5

GTTACCTAAA TGAGCGATAG AGTGCTTCGG TAGTAAAAAT ATCTTTCAGG AAGTAAACAC 6

ATCAGGAGCG ATAGCGGTGA ATTATTCGTG GTTTTGTCGA TTCGGCATAG TGGCGATAAC 6

TGAATGCCGG ATCGGTACTG CAGGTGTTTA AACACACCGT AAATAATAAG TAGTATTAAG 7

GAGTTGTT 7

ATG AAA AAT ATT ATT TTA TCC ACT TTA GTT ATT ACT ACA AGC GTT TTG 7
Met Lys Asn Ile Ile Leu Ser Thr Leu Val Ile Thr Thr Ser Val Leu
                5                   10                  15

GTT GTA AAT GTT GCA CAG GCC GAT ACT AAC GCC TTT TCC GTG GGG TAT 8
Val Val Asn Val Ala Gln Ala Asp Thr Asn Ala Phe Ser Val Gly Tyr
            20                  25                  30

GCA CGG TAT GCA CAA AGT AAA GTT CAG GAT TTC AAA AAT ATC CGA GGG 8
Ala Arg Tyr Ala Gln Ser Lys Val Gln Asp Phe Lys Asn Ile Arg Gly
        35                  40                  45

GTA AAT GTG AAA TAC CGT TAT GAG GAT GAC TCT CCG GTA AGT TTT ATT 9
Val Asn Val Lys Tyr Arg Tyr Glu Asp Asp Ser Pro Val Ser Phe Ile
    50                  55                  60

TCC TCG CTA AGT TAC TTA TAT GGA GAC AGA CAG GCT TCC GGG TCT GTT 9
Ser Ser Leu Ser Tyr Leu Tyr Gly Asp Arg Gln Ala Ser Gly Ser Val
65                  70                  75                  80

GAG CCT GAA GGT ATT CAT TAC CAT GAC AAG TTT GAG GTG AAG TAC GGT 10
Glu Pro Glu Gly Ile His Tyr His Asp Lys Phe Glu Val Lys Try Gly
85 90 95

TCT TTA ATG GTT GGG CCA GCC TAT CGA TTG TCT GAC AAT TTT TCG TTA 10
Ser Leu Met Val Gly Pro Ala Tyr Arg Leu Ser Asp Asn Phe Ser Leu
100 105 110

TAC GCG CTG GCG GGT GTC GGC ACG GTA AAG GCG ACA TTT AAA GAA CAT 11
Tyr Ala Leu Ala Gly Val Gly Thr Val Lys Ala Thr Phe Lys Glu His
115 120 125

TCC ACT CAG GAT GGC GAT TCT TTT TCT AAC AAA ATT TCC TCA AGG AAA 11
Ser Thr Gln Asp Gly Asp Ser Phe Ser Asn Lys Ile Ser Ser Arg Lys
130 135 140

ACG GGA TTT GCC TGG GGC GCG GGT GTA CAG ATG AAT CCG CTG GAG AAT 12
Thr Gly Phe Ala Trp Gly Ala Gly Val Gln Met Asn Pro Leu Glu Asn
145 150 155 160

ATC GTC GTC GAT GTT GGG TAT GAA GGA AGC AAC ATC TCC TCT ACA AAA 12
Ile Val Val Asp Val Gly Tyr Glu Gly Ser Asn Ile Ser Ser Thr Lys
165 170 175

ATA AAC GGC TTC AAC GTC GGG GTT GGA TAC CGT TTC TGA AAGC 13
Ile Asn Gly Phe Asn Val Gly Val Gly Tyr Arg Phe
180 185

ATAAGCTATG CGGAAGGTTC GCCTTCCGCA CCGCCAGTCA ATAAAACAGG GCTTCTTTAC 13
CAGTGACACG TACCTGCCTG TCTTTTCTCT CTTCGTCATA CTCTCTTCGT CATAGTGACG 14
CTGTACATAA CATCTCACTA GCATAAGCAC AGATAAAGGA TTGTGGTAAG CAATCAAGGT 14
TGCTCAGGTA GGTGATAAGC AGGAAGGAAA ATCTGGTGTA AATAACGCCA GATCTCACAA 15
GATTCACTCT GAAAAATTTT CCTGGAATTA ATCACAATGT CATCAAGATT TTGTGACCGC 16
CTTCGCATAT TGTACCTGCC GCTGAACGAC TACTGAAAAG TAGCAAGGTA TGTATTTTAT 16
CCAGGAGAGC ACCTTTTTTG CGCCTGGCAG AAGTCCCCAG CCGCCACTAG CTCAGCTGGA 17
TAGAGCATCA ACCTCCTAAG TTGATGGTGC GAGGTTCGAG GCCTCGGTGG CGGTCCAATG 17
TGGTTATCGT ATAATGTTAT TACCTCAGTG TCAGGCTGAT GATGTGGGTT CGACTCCCAC 18
TGACCACTTC AGTTTTGAAT AAGTATTGTC TCGCAACCCT GTTACAGAAT AATTTCATTT 19
ATTACGTGAC AAGATAGTCA TTTATAAAAA ATGCACAAAA ATGTTATTGT CTTTTATTAC 19
TTGTGAGTTG TAGATTTTTC TTATGCGGTG AATCCCCCTT TGCGGCGGGG CGTCCAGTCA 20
AATAGTTAAT GTTCCTCGCG AACCATATTG ACTGTGGTAT GGTTCACCGG GAGGCACCCG 20
GCACCGCAAT TTTTTATAAA ATGAAATTCA CACCCTATGG TTCAGAGCGG TGTCTTTTTA 21
CATCAGGTGG GCAAGCATAA TGCAGGTTAA CTTGAAAGAT ACGATCAATA GCAGAAACCA 22
GTGATTTCGT TTATGGCCTG GGGATTTAAC CGCGCCAGAG CGTATGCAAG ACCCTGGCGC 22
GGTTGGCCGG TGATCGTTCA ATAGTGCGAA TATGAATGGT TACCAGCCGC CTGCGAATTC 23

(2) INFORMATION FOR SEQ ID NO: 2:

(i) SEQUENCE CHARACTERISTICS:

(A) LENGTH: 53
(B) TYPE: nucleic acid
(C) STRANDEDNESS: single
(D) TOPOLOGY: linear

(xi) SEQUENCE DESCRIPTION: SEQ ID NO: 2:

CATTTCTCAT TGATAATGAG AATCATTATT GACATAATTG TTATTATTTT ACG

## Description

The invention relates to *Salmonella* vaccines.

Enteric fevers and diarrheal diseases, e.g., typhoid fever and cholera, are major causes of morbidity and mortality throughout the developing world, Hook et al., 1980, In Harrison's Principles of Internal Medicine, 9th Ed., 641-848, McGraw Hill, New York. Traditional approaches to the development of vaccines for bacterial diseases include the parenteral injection of purified components or killed organisms. These parenterally administered vaccines require technologically advanced preparation, are relatively expensive, and are often, because of dislike for needle-based injections, resisted by patients. Live oral vaccine strains have several advantages over parenteral vaccines: low cost, ease of administration, and simple preparation.

The development of live vaccines has often been limited by a lack of understanding of the pathogenesis of the disease of interest on a molecular level. Candidate live vaccine strains require nonrevertible genetic alterations that affect the virulence of the organism, but not its induction of an immune response. Work defining the mechanisms of toxigenesis of *vibrio cholerae* has made it possible to create live vaccine strains based on deletion of the toxin genes, Mekalanos et al., 1983, Nature 306:551, Levine et al., 1988, Infect. Immun. 56:161.

Recent studies have begun to define the molecular basis of *Salmonella typhimurium* macrophage survival and virulence, Miller et al., 1989, Proc. Natl. Acad. Sci. USA 86:5054, hereby incorporated by reference. *Salmonella typhimurium* strains with mutations in the positive regulatory regulon *phoP* are markedly attenuated in virulence for BALB/c mice. The *phoP* regulon is composed of two genes present in an operon, termed *phoP* and *phoQ*. The *phoP* and *phoQ* gene products are highly similar to other members of bacterial two-component transcriptional regulators that respond to environmental stimuli and control the expression of a large number of other genes. A mutation at one of these *phoP* regulatory region regulated genes, *pagC*, confers a virulence defect. Strains with *pagC*, *phoP*, or *phoQ* mutations afford partial protection to subsequent challenge by wild-type *S*. *typhimurium*.

Salmonella species cause a spectrum of clinical disease that includes enteric fevers and acute gastroenteritis, Hook et al., 1980, *supra*. Infections with *Salmonella* species are more common in immunosuppressed persons, Celum et al., 1987, J. Infect. Dis. 156:998. *S*. *typhi*, the bacterium that causes typhoid fever, can only infect man, Hook et al., 1980, *supra*. The narrow host specificity of *S*. *typhi* has resulted in the extensive use of *S*. *enteriditis typhimurium* infection of mice as a laboratory model of typhoid fever, Carter et al., 1984 J. Exp. Med. 139:1189. *S. typhimurium* infects a wider range of hosts, causing acute gastroenteritis in man and a disease similar to typhoid fever in the mouse and cow.

*Salmonella* infections are acquired by oral ingestion. The organisms, after traversing the stomach, replicate in the small bowel, Hornik et al., 1970, N. Eng. J. Med. 283:686. *Salmonella* are capable of invasion of the intestinal mucosal cells, and *S. typhi* can pass through this mucosal barrier and spread via the Peyer's patches to the lamina propria and regional lymph nodes. Colonization of the reticuloendothelial cells of the host then occurs after bacteremia. The ability of *S. typhi* to survive and replicate within the cells of the human reticuloendothelial system is essential to its pathogenesis, Hook et al., 1980, *supra*, Hornick et al., 1970, *supra*, and Carter et al., 1984, *supra*.

Immunity to *Salmonella typhi* involves humoral and cell-mediated immunity, Murphy et al., 1987, J. Infect. Dis. 156:1005, and is obtainable by vaccination, Edelman et al., 1986, Rev. Inf. Dis. 8:324. Recently, human field trials demonstrated significant protective efficacy against *S. typhi* infection after intramuscular vaccination with partially purified Vi antigen, Lanata et al., 1983, Lancet 2:441. Antibody-dependent enhancement of *S. typhi* killing by T cells has been demonstrated in individuals who received a live *S. typhi* vaccine, indicating that these antibodies may be necessary for the host to generate a cell-mediated immune response, Levine et al., 1987, J. Clin. Invest. 79:888. The cell-mediated immune response is important in typhoid immunity since killed vaccines that do not induce this immune response are not protective in man, Collins et al., 1972, Infect. Immun. 41:742.

We are concerned with a vaccine, preferably a live vaccine, including a *Salmonella* cell, e.g., a *S. typhi*, *S. enteritidis typhimurium*, or *S. cholerae-suis* cell, the virulence of which is attenuated by the constitutive expression of a gene under the control of a two-component regulatory system. In preferred embodiments the constitutive expression is the result of a mutation at a component of the two-component regulatory system. In preferred embodiments the bacterial cell includes a second mutation which attenuates virulence.

In yet other preferred embodiments of the vaccine the two-component regulatory system is the phoP regulatory region, and the gene under the control of the two-component system is a phoP regulatory region regulated gene, e.g., a *prg* or *pag* gene, e.g., *pagC*. In preferred embodiments constitutive expression is the result of a change or mutation (preferably a non-revertible mutation) at the promoter of the regulated gene or of the phoP regulatory region, e.g., a mutation in the *pho*Q or the *pho*P gene, e.g., the *pho*P^{c} mutation.

In preferred embodiments of the vaccine the *Salmonella* cell includes a first mutation which attenuates virulence, e.g., a mutation in a *pho*P regulatory region gene, e.g., a mutation in the *pho*P or *pho*Q gene, e.g., *pho*P^{c}, or a mutation in a phoP regulatory region regulated gene, and a second mutation which attenuates virulence, a mutation in a phoP regulatory region regulated gene, e.g., a mutation in a *prg* or *pag* locus, e.g., a *pagC* mutation.

In yet another preferred embodiment of the vaccine, the two-component regulatory system is the phoP regulatory region, and the gene under its control is a *prg* or a *pag* gene, e.g., the *pagC* gene.

In preferred embodiments the *Salmonella* cell expresses a phoP regulatory region regulated gene constitutively (the constitutive expression preferably caused by a mutation, preferably a non-revertible mutation, e.g., a deletion in the phoP regulatory region, e.g., a mutation in the *phoQ* or *phoP* gene, e.g., phoP^{c}), and which includes a virulence attenuating mutation, preferably a non-revertible mutation, e.g., a deletion in a phoP regulatory region regulated gene, e.g., a *prg* or *pag* gene, e.g., *pagC* which does not result in the constitutive expression of a gene under the control of the phoP regulatory region.

We are also concerned with a *Salmonella* cell, e.g., a *S. typhi* cell, an *S. enteritidis typhimurium* or a *S. cholerae-suis* cell, including a virulence attenuating mutation in a gene regulated by a two-component regulatory system. The virulence attenuating mutation is in a phoP regulatory region regulated gene namely, a *prg* or *pag* gene, e.g., *pagC*.

In preferred embodiments the bacterial cell includes a second mutation, e.g., in an aromatic amino acid synthetic gene, e.g., an *aro* gene, in a phoP regulatory region gene, e.g., the phoP or phoQ genes, or in a phoP regulating region regulated gene, e.g., a *prg* or a *pag* gene, e.g., *pag*C, which attenuates virulence but which does not result in constitutive expression of a phoP regulatory region regulated gene.

We are also concerned with a live *Salmonella* cell, or a substantially purified preparation thereof, e.g., a *S. typhi*, *S. enteriditis typhimurium*, or *S. cholerae-suis* cell, in which there is inserted into a virulence gene, specifically a phoP regulating region regulated gene, namely a *prg* or a *pag* locus, e.g., *pagC*, a gene encoding a heterologous protein, or a heterologous regulatory element.

In preferred embodiments the live *Salmonella* cell carries a second mutation, e.g., an *aro* mutation, e.g., an *aro*A mutation, e.g., *aro*A⁻ or *aro*ADEL407, that further attenuates virulence.

In preferred embodiments the DNA encoding a heterologous protein is under the control of an environmentally regulated promoter. In other preferred embodiments the live *Salmonella* cell further includes a DNA sequence encoding T7 polymerase under the control of an environmentally regulated promoter and a T7 transcriptionally sensitive promoter, the T7 transcriptionally sensitive promoter controlling the expression of the heterologous antigen.

We are also concerned with a vector capable of integrating into the chromosome of *Salmonella* including: a first DNA sequence encoding a heterologous protein; a second (optional) DNA sequence encoding a marker e.g., a selective marker, e.g., a gene that confers resistance for a heavy metal resistance or a gene that compliments an aurotrophic mutation carried by the strain to be transformed; and a third DNA sequence, e.g., a *pho*P regulon encoded gene, e.g., a *prg* or a *pag* locus, e.g., *pag*C, encoding a product necessary for virulence, the third DNA sequence being mutationally inactivated.

In other preferred embodiments: the first DNA sequence is disposed on the vector so as to mutationally inactivate the third DNA sequence; the vector cannot replicate in a wild-type *Salmonella* strain; the heterologous protein is under the control of an environmentally regulated promoter; and the vector further includes a DNA sequence encoding T7 polymerase under the control of an environmentally regulated promoter and a T7 transcriptionally sensitive promoter, the T7 transcriptionally sensitive promoter controlling the expression of the heterologous antigen.

According to a first aspect of the present invention, we provide a vaccine comprising a *Salmonella* cell the virulence of which is attenuated by a first mutation in the *phoP* regulatory region causing constitutive expression of a gene under the control of said region and by a second mutation at a *pag* or *prg* gene, neither mutation comprising the insertion of an antibiotic resistance gene.

The invention provides, in a second and alternative aspect thereof, a vaccine comprising a *Salmonella* cell the virulence of which is attenuated by a mutation in a *pag* or a *prg* gene, the mutation not comprising the insertion of an antibiotic resistance gene.

In a third alternative aspect of this invention, there is provided a *Salmonella* cell which constitutively expresses a *phoP* regulatory region regulated gene and which comprises a virulence attenuating mutation in a *prg* or a *pag* gene, the virulence attenuating mutation not comprising the insertion of an antibiotic resistance gene.

There is provided, in a fourth alternative aspect of this invention, a *Salmonella* cell which comprises a virulence attenuating mutation in a *pag* or a *prg* gene, the virulence attenuating mutation not comprising the insertion of an antibiotic resistance gene.

We provide, according to a fifth alternative aspect of this invention, a live *Salmonella* cell into a *pag* or a *prg* gene of which is inserted DNA encoding a heterologous protein or a regulatory element of the gene for the heterologous protein, whereby the virulence of said cell is attenuated by said insertion, the DNA not comprising an antibiotic resistance gene.

According to a sixth and further alternative aspect of this invention, we provide a vector capable of integrating into the chromosome of *Salmonella* comprising a first DNA sequence encoding a heterologous protein, a second DNA sequence encoding a marker, and a third DNA sequence encoding mutationally inactivated *Salmonella* virulence factor, said virulence factor being encoded by a *pag* or *prg* gene which has been mutationally inactivated otherwise than by insertion of an antibiotic resistance gene.

The term "two-component regulatory system", as used herein, refers to a bacterial regulatory system that controls the expression of multiple proteins in response to environmental signals. The two-components referred to in the term are a sensor, which may, e.g., sense an environmental parameter and in response thereto promote the activation, e.g. by promoting the phosphorylation, of the second component, the activator. The activator affects the expression of genes under the control of the two-component system. A two-component system can include, e.g., a histidine protein kinase and a phosphorylated response regulator, as is seen in both gram positive and gram negative bacteria. In E. coli, e.g., 10 kinases and 11 response regulators have been identified. They control chemotaxis, nitrogen regulation, phosphate regulation, osmoregulation, sporulation, and many other cellular functions, Stock et al., 1989 Microbiol. Rev. 53:450-490, hereby incorporated by reference. A two-component system also controls the virulence of *Agrobacterium tumefasciens* plant tumor formation, Leroux et al. EMBO J 6:849-856, hereby incorporated by reference). Similar virulence regulators are involved in the virulence of *Bordetella pertussis* Arico et al., 1989, Proc. Natl. Acad. Sci. USA 86:6671-6675, hereby incorporated by reference, and *Shigella flexneri*, Bernardini et al., 1990, J. Bact. 172:6274-6281, hereby incorporated by reference.

Environmentally regulated, as used herein refers to a pattern of expression wherein the expression of a gene in a cell depends on the levels of some characteristic or component of the environment in which the cell resides. Examples include promoters in biosynthetic pathways which are turned on or off by the level of a specific component or components, e.g., iron, temperature responsive promoters, or promoters which are expressed more actively in specific cellular compartments, e.g., in macrophages or vacuoles.

A vaccine, as used herein, is a preparation including materials that evoke a desired biological response, e.g., an immune response, in combination with a suitable carrier. The vaccine may include live organism, in which case it is usually administered orally, or killed organisms or components thereof, in which case it is usually administered perinterally. The cells used for the vaccine of the invention are preferably alive and thus capable of colonizing the intestines of the inoculated animal.

A mutation, as used herein, is any change (in comparison with the appropriate parental strain) in the DNA sequence of an organism. These changes can arise e.g., spontaneously, by chemical, energy e.g., X-ray, or other forms of mutagenesis, by genetic engineering, or as a result of mating or other forms of exchange of genetic information. Mutations include e.g., base changes, deletions, insertions, inversions, translocations or duplications.

A mutation attenuates virulence it, as a result of the mutation, the level of virulence of the mutant cell is decreased in comparison with the level in a cell of the parental strain, as measured by (a) a significant (e.g., at least 50%) decrease in virulence in the mutant strain compared to the parental strain, or (b) a significant (e.g., at least 50%) decrease in the amount of the polypeptide identified as the virulence factor in the mutant strain compared to the parental strain.

A non-revertible mutation, as used herein, is a mutation which cannot revert by a single base pair change, e.g., deletion or insertion mutations and mutations that include more than one lesion, e.g., a mutation composed of two separate point mutations.

The phoP regulatory region, as used herein, is a two-component regulatory system that controls the expression of *pag* and *prg* genes. It includes the phoP locus and the phoQ locus.

phoP regulatory region regulated genes, as used herein, refer to genes such as *pag* and *prg* genes.

*pag*, as used herein, refers to a gene which is positively regulated by the phoP regulon.

*prg*, as used herein, refers to a gene which is negatively regulated by the phoP regulon.

An aromatic amino acid synthetic gene, as used herein, is a gene which encodes an enzyme which catalyzes a step in the synthesis of an aromatic amino acid. *aro*A, *aro*C, and *aro*D are examples of such genes in *Salmonella*.

Mutations in these genes can attenuate virulence without the total loss of immunogenicity. It should be noted that arrangements only with an *aro* mutation and not also with a mutation in the *prg* or *pag* genes are not within the scope of the present invention.

Abnormal expressions, as used herein, means expression which is higher or lower than that seen in wild type.

Heterologous protein, as used herein, is a protein that in wild type, is not expressed or is expressed from a different chromosomal site, e.g., a heterologous protein is one encoded by a gene that has been inserted into a second gene.

Virulence gene, as used herein, is a gene the inactivation of which results in a *Salmonella* cell with less virulence than that of a similar *Salmonella* cell in which the gene is not inactivated. Examples include the phoP and *pag*C genes.

A marker, as used herein, is gene product the presence of which is easily determined, e.g., a gene product that confers resistance to a heavy metal or a gene product which allows or inhibits growth under a given set of conditions.

Purified preparation, as used herein, is a preparation, e.g., of a protein, which is purified from the proteins, lipids, and other material with which it is associated. The preparation is preferably at least 2-10 fold purified.

Constitutive expression, as used herein, refers to gene expression which is modulated or regulated to a lesser extent than the expression of the same gene in an appropriate control strain, e.g., a parental or in wild-type strain. For example, if a gene is normally repressed under a first set of conditions and derepressed under a second set of conditions constitutive expression would be expression at the same level, e.g., the repressed level, the derepressed level, or an intermediate level, regardless of conditions. Partial constitutive expression is included within the definition of constitutive expression and occurs when the difference between two levels of expression is reduced in comparison in what is seen in an appropriate control strain, e.g., a wild-type or parental strain.

A substantially purified preparation of a bacterial cell is a preparation of cells wherein contaminating cells without the desired mutant genotype constitute less than 10%, preferably less than 1%, and more preferably less than 0.1% of the total number of cells in the preparation.

We describe herein the attenuation of virulence *Salmonella* and of vaccines that include *Salmonella*, especially vaccines that include live *Salmonella*, by mutations in two-component regulatory systems and/or in genes regulated by these systems. Our vaccines are highly attenuated for virulence but retain immunogenicity, thus they are both safe and effective.

Our vectors allow the rapid construction of strains containing DNA encoding heterologous proteins, e.g., antigens. The heterologous protein encoding DNA is chromosomally integrated, and thus stable, unlike plasmid systems which are dependent on antibiotic resistance or other selection pressure for stability. We describe live *Salmonella* cells in which the expression of heterologous protein is under the control of an environmentally responsive promoter do not express the heterologous protein at times when such expression would be undesirable e.g., during culture, vaccine preparation, or storage, contributing to the viability of the cells, but when administered to humans or animals, express large amounts of the protein. This is desirable because high expression of many heterologous proteins in *Salmonella* can be associated with toxicity to the bacterium. The use of only a single integrated copy of the DNA encoding the heterologous protein also contributes to minimal expression of the heterologous protein at times when expression is not desired. In embodiments where a virulence gene, e.g., the *pag*C gene, contains the site of integration for the DNA encoding the heterologous protein the virulence of the organism is attenuated.

Other features and advantages will be apparent from the following description of preferred embodiments.

In the drawings:-
Fig. 1 is a graph of the survival of Salmonella strains within macrophages.
Fig. 2 is a map of the restriction endonuclease sites of the *pag*C locus.
Fig. 3 is a map of the DNA sequence of the *pag* C region (Sequence ID No. 1).

### Strain Deposit

*PhoP*^{c} strain CS022 (described below) has been deposited with the American Type Culture Collection (Rockville, MD) and has received ATCC designation

### Constitutive Expression of the PhoP Regulon Attenuates Salmonella Virulence and Survival within Macrophages

The *phoP* constitutive allele (*PhoP*^{c}), *pho*-*24*, results in derepression of *pag* loci. Using diethyl sulfate mutagenesis of *S. typhimurium* LT-2, Ames and co-workers isolated strain TA2367 *pho*-*24* (all strains, materials, and methods referred to in this section are described below), which contained a *phoP* locus mutation that resulted in constitutive production of acid phosphatase in rich media, Kier et al., 1979, J. Bacteriol. 138:155, hereby incorporated by reference. This *phoP*-regulated acid phosphatase is encoded by the *phoN* gene, a *pag* locus, Kier et al., 1979, supra, Miller et al., 1989, supra. To analyze whether the *pho*-*24* allele increased the expression of other *pag* loci the effect of the *pho-24* allele on the expression of other *pag* loci recently identified as transcriptional (e.g., *pag*A and *pag*B) and translational (e.g., *pag*C) fusion proteins that required *pho*P and *pho*Q for expression, Miller et al., 1989, supra, was determined. *pag* gene fusion strains, isogenic except for the *pho-24* allele, were constructed and assayed for fusion protein activity. PhoP^{c} derivatives of the *pag*A::Mu dJ and *pag*B::Mu dJ strains produced 480 and 980 U, respectively, of β-galactosidase in rich medium, an increase of 9- to 10-fold over values for the fusion strains with a wild-type *phoP* locus, see Table 1.

**TABLE 1**

| Bacterial strains and properties | | | |
|---|---|---|---|
| Strain | Genotype | Enzyme activity (U)^{a} | Reference or source |
| 10428 | Wild type | 180 (A) | ATCC; Miller et al., 1989, supra |
| TA2367 | *pho*-*24* | 1,925 (A) | Kier et al., 1974, supra |
| CS003 | Δ*phoP* Δ*purB* | <10 (A) | Miller et al., 1989, supra |
| CS022 | *pho-24* | 1,750 (A) | This work |
| CS023 | *pho-24 phoN2* | 25 (A) | This work |
| | *zxx*::6251Tn10d-Cam | | |
| CS012 | *pag*A1::MU dJ | 45 (B) | Miller et al., 1989, supra |
| CS013 | *pag*B1::MU dJ | 120 (B) | Miller et al., 1989, supra |
| CS119 | *pag*C1::Tn*phoA phoN2* | 85 (C) | Miller et al., 1989, supra |
| | *zxx*::6251Tn*10*d-Cam | | |
| SC024 | *pag*A1::Mu dJ *pho-24* | 450 (B) | This work |
| SC025 | *pag*B1::Mu dJ *pho-24* | 980 (B) | This work |
| SC026 | *pag*Cl::Tn*phoApho*-*24phoN2* | 385 (B) | This work |
| | *zxx*::6251Tn*10*d-Cam | | |
| CS015 | *phoP102*::Tn*10*d-Cam | <10 (A) | Miller et al., 1989, supra |
| TT13208 | *phoP105*::Tn*10*d | <10 (A) | --^{b} |

| | | | |
|---|---|---|---|
| ^{a} A. Acid phosphatase; B, β-galactosidase; C, alkaline phosphatase. | | | |
| ^{b} Gift of Ning Zhu and John Roth. | | | |

The *pag*C::Tn*pho*A gene fusion produced 350 U of alkaline phosphatase, an increase of three- to fourfold over that produced in strain CS119, which is isogenic except for the *pho*-*24* mutation, Miller et al., 1989, supra. These results compare with a ninefold increase in the acid phosphatase activity in strain CS022 on introduction of the *pho-24* allele. Therefore, these available assays for *pag* gene expression document that the *pho-24* mutation causes constitutive expression of *pag* loci other than *phoN*.

Identifications of protein species that are repressed as well as activated in the PhoP^{c} mutant strain Whole-cell proteins of strain CS022 were analyzed to estimate the number of protein species that could be potentially regulated by the PhoP regulon. Remarkably, analysis by one-dimensional polyacrylamide gel electrophoresis of the proteins produced by strains with the PhoP^{c} phenotype indicated that some protein species were decreased in expression when many presumptive *pag* gene products were fully induced by the *pho-24* mutation. The proteins decreased in the PhoP^{c} strain might represent products of genes that are repressed by the PhoP regulator. Genes encoding proteins decreased by the *pho-24* allele are designated *prg* loci, for *phoP*-repressed genes. Comparison of wild-type, PhoP⁻, and PhoP^{c} mutant strain proteins shows that growth in LB medium at 37°C represents repressing conditions for *pag* gene products and derepressing conditions for *prg* gene products.

To estimate the total number of potentially PhoP-regulated gene products, the total cell proteins of wild-type and PhoP^{c} mutant strains grown in LB were analyzed by two-dimensional gel electrophoresis. At least 40 species underwent major fluctuation in expression in response to the *pho*-*24* mutation.

Virulence defects of the PhoP^{c} strain Remarkably, strains with the single *pho-24* mutation were markedly attenuated for virulence in mice (Table 2). The number of PhoP^{c} organisms (2 x 10⁵) that killed 50% of BALB/c mice challenged (LD₅₀) by the intraperitoneal (i.p.) route was near that (6 x 10⁵) of PhoP⁻ bacteria, Miller et al., 1989, supra. The PhoP^{c} strains had growth comparable to wild-type organisms in rich and minimal media. The PhoP^{c} mutants were also tested for alterations in lipopolysaccharide, which could explain the virulence defect observed. Strain CS022 had normal sensitivity to phage P22, normal group B reactivity to antibody to O antigen, and a lipopolysaccharide profile identical to that of the parent strain, as determined by polyacrylamide gel electrophoresis and staining.

**Table 2**

| Virulence and protective efficacy of PhoP^{c} and PhoP⁻ *Salmonella* strains | | | | | |
|---|---|---|---|---|---|
| Immunizing dose | No. of initial survivors/total | No. of survivors/total after wild-type challenge dose of: | | | |
| | | 5x10⁷ | 5x10⁵ | 5x10⁴ | 5x10³ |
| PhoP^{c} organisms | | | | | |
| 15 | 13/13 | | 5/5 | 4/5 | |
| 50 | 4/4 | | | | 4/4 |
| 1.5x10² | 11/11 | | 4/4 | 3/3 | |
| 5x10² | 16/16 | | | | 4/4 |
| 1.5x10³ | 5/5 | | 3/3 | 2/2 | 4/4 |
| 5x10³ | 4/4 | | | | 4/4 |
| 1.5x10⁴ | 5/5 | | 3/3 | 2/2 | |
| 5x10⁴ | 19/23 | | | | 4/4 |
| 1.5x10⁵ | 5/5 | | 3/3 | 2/2 | |
| 5x10⁵ | 1/4 | | | | 1/1 |
| 5x10⁶ | 0/6 | | | | |
| 3x10⁹(*) | 5/5 | 5/5 | | | |
| 3x10¹⁰^{(*)} | 5/5 | 5/5 | | | |
| 1.5x10¹¹(*) | 5/5 | 5/5 | | | |

| Pho^{P-}organisms | | | | | |
|---|---|---|---|---|---|
| 6x10³ | 36/36 | | 0/12 | 0/12 | 0/12 |
| 6x10⁴ | 36/36 | | 0/12 | 0/12 | 3/12 |
| 6x10⁵ | 19/36 | | 0/6 | 0/6 | 4/7 |
| 5x10¹⁰(*) | 7/7 | 3/7 | | | |

| | | | | | |
|---|---|---|---|---|---|
| (*) Organisms were administered by the oral route. In all other experiments, organisms were administered by i.p. challenge. | | | | | |

Since the TA2367 *pho-24* strain was constructed by chemical mutagenesis and could have another linked mutation responsible for its virulence defect revertants of the PhoP^{c} were isolated to determine whether the *pho*-*24* allele was responsible for the attenuation of virulence observed. Phenotype PhoP^{c} revertants, identified by the normal levels of acid phosphatase in rich medium, were isolated among the bacteria recovered from the livers of mice infected with strain CS022. Six separate phenotypic revertants, designated CS122 to CS128, were found to be fully virulent (LD₅₀ of less than 20 organisms for BALB/c mice). The locus responsible for the reversion phenotype was mapped in all six revertants tested for virulence by bacteriophage P22 cotransduction and had linkage characteristics consistent with the *phoP* locus (greater than 90% linkage to *purB*). These data indicate that these reversion mutations are not extragenic suppressors but are intragenic suppressors or true revertants of the *pho-24* mutation. Thus, the virulence defect of PhoP^{c} mutants is probably the result of a single revertible mutation in the *phoP* locus and not the result of a second unrelated mutation acquired during mutagenesis.

Reversion frequency of the PhoP^{c} phenotype The reversion frequency of the PhoP^{c} mutation *in vivo* in mice was investigated to assess whether reversion could reduce the LD₅₀ of this strain. The presence of the revertants of strain CS022 was tested for by administering 10⁶, 10⁴, and 10² challenge organisms to each of eight animals by i.p. injection. On day 7, three animals died that received 10⁶ PhoP^{c} organisms. On that day, the livers and spleens of all animals were harvested and homogenized in saline. After appropriate dilution, 10% of the tissue was plated on LB plates containing the chromogenic phosphatase substrate XP. Revertants were identified by their lighter blue colonies compared with PhoP^{c} bacteria and were confined by quantitative acid phosphatase assays. An estimated 10⁷, 10⁵, and 10³ organisms per organ were recovered from animals at each of the three respective challenge doses. Revertants were identified only at the highest dose and comprised 0.5 to 1%, or 10⁵ organisms per organ, at the time of death. It is likely that revertants are able to compete more effectively for growth in these macrophage-containing organs, since strain CS022 is deficient in survival within macrophages (see below). However, revertants were not identified if fewer than 10⁵ organisms were administered in the challenge dose, suggesting that the reversion frequency must be approximately 10⁻⁵. The reversion rate of the PhoP^{c} phenotype for CS022 bacteria grown in LB is in fact 6x10⁻⁴ when scored by the same colony phenotypes. The percentage of revertants recovered from animals near death suggests that pressure is applied *in vivo* that selects for revertants of the PhoP^{c} phenotype and implies that the virulence defect observed could be much greater quantitatively for a strain with a nonrevertible PhoP^{c} mutation.

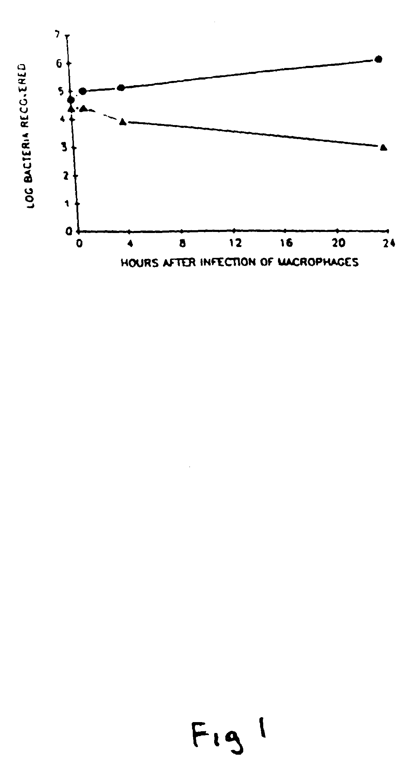

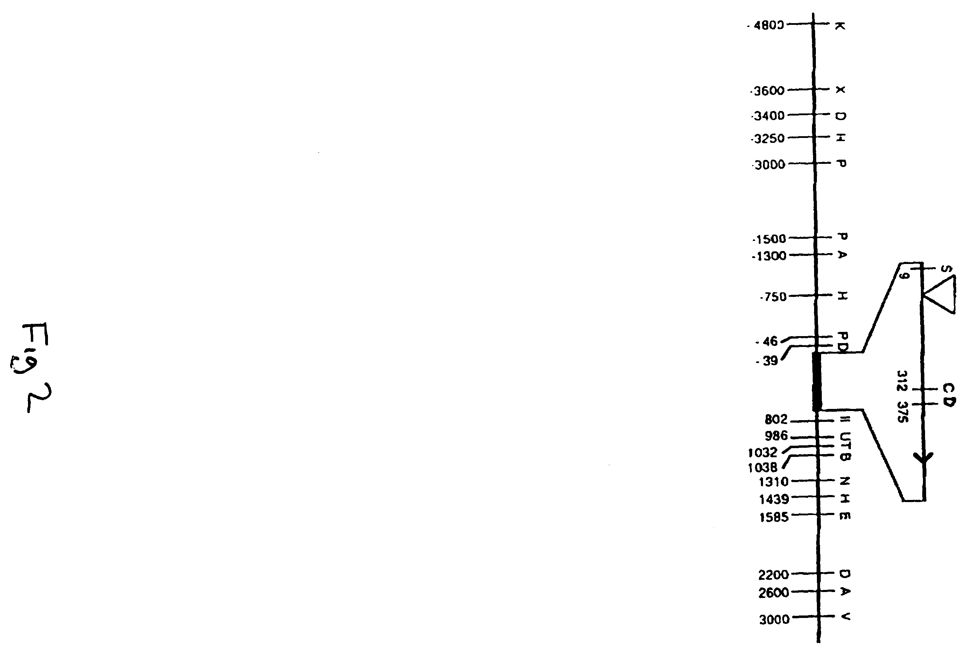

The PhoP^{c} strain is deficient in survival within macrophages Because of the importance of survival within macrophages to *Salmonella* virulence Fields et al., 1986, Proc. Natl. Acad. Sci. USA 83:5189, hereby incorporated by reference, PhoP^{c} bacteria were tested for this property. Strain CS022 was defective in the ability to grow and persist in macrophages as compared with wild-type organisms (Fig. 1). In Fig. 1 the survival of strain CS022 (PhoP^{c}) (triangles) in cultured macrophages is compared with that of wild-type *S. typhimurium* ATCC 10428 (cicles). The experiment shown is a representative one. The difference between the two strains at 4 and 24 hours is significant (*P* < 0.05). PhoP⁻ bacteria seemed to have a macrophage survival defect qualitatively similar to that of PhoP^{c} bacteria but survived consistently better by two- to threefold in side-by-side experiments. The increased recovery of organisms that reverted to PhoP^{c} phenotype in mouse organs rich in macrophage content is consistent with the reduced macrophage survival of PhoP^{c} mutants in vitro.

Use of the PhoP^{c} strain as a live vaccine It has been previously reported that PhoP⁻ strains are useful as live vaccines in protecting against mouse typhoid, Miller et al., 1989, supra. The immunogenicity of PhoP^{c} when used as live attenuated vaccines in mice was compared with the of PhoP⁻. This was done by simultaneous determination of survival, after graded challenge doses with the wild-type strain ATCC 10428, in mice previously immunized with graded doses of the two live vaccine strains. CS015 *phoP*::Tn*10*d-Cam and CS022 *pho-24*, as well as a saline control. The results obtained (Table 2) suggest the following conclusions: (i) small i.p. doses of the PhoP^{c} strain (e.g., 15 organisms) effectively protect mice from challenge doses as large as 5x10⁵ bacteria (a challenge dose that represents greater than 10⁴ i.p. LD₅₀s), (ii) large doses of PhoP^{c} organisms given orally completely protect mice from an oral challenge consisting of 5x10⁷ wild-type bacteria (over 200 oral wild-type LD₅₀s) and (iii) by comparison, a large dose of PhoP⁻ organisms (5x10⁵) does not provide similar protection. The reversion of the PhoP^{c} mutation in vivo somewhat complicates the analysis of the use of these strains as vaccines, since revertants of the CS022 strain (i.e., wild-type cells) could increase immunogenicity). However, we were unable to identify revertants by examining 10% of the available spleen and liver tissue from those mice that received 10⁴ or fewer organisms.

Strains, Materials and Methods The strains, materials, and methods used in the PhoP regulon work described above are as follows.

American Type Culture Collection (ATCC) strain 14028, a smooth virulent strain of *S. typhimurium*, was the parent strain for all virulence studies. Strain TT13208 was a gift from Nang Zhu and John Roth. Strain TA2367 was a generous gift of Gigi Stortz and Bruce Ames, Kier et al., 1979, supra. Bacteriophage P22HT *int* was used in transductional crosses to construct strains isogenic except for *phoP* locus mutations, Davis et al., 1980, Advanced Bacterial Genetics, p. 78, 87. Cold Spring Harbor Laboratory, Cold Spring Harbor, NY, hereby incorporated by reference. Luria broth was used as rich medium, and minimal medium was M9, Davis et al., 1980, supra. The chromogenic phosphatase substrate 5-bromo-4-chloro-3indolyl phosphate (XP) was used to qualitatively access acid and alkaline phosphatase production in solid media.

Derivatives of *S. typhimurium* ATCC 10428 with the *pho-24* mutation were constructed by use of strain TA2367 as a donor of the *purB* gene in a P22 transductional cross with strain CS003 Δ*phoP* Δ*purB*, Miller et al., 1989, supra. Colonies were then selected for the ability to grow on minimal medium. A transductant designated CS022 (phenotype PhoP^{c}) that synthesized 1,750 U of acid phosphatase in rich medium (a ninefold increase over the wild-type level in rich medium) was used in further studies.

Derivatives of strains CS022 and CS023 *pho-24 phoN2 zxx*::6251Tn*10*d-Cam, and acid phosphatase-negative derivative of CS022, containing *pag* gene fusions were constructed by bacteriophage P22 transductional crosses, using selection of Tn*phoA*- or Mu dJ-encoded kanamycin resistance. Strains were checked for the intact *pag* gene fusion by demonstration of appropriate loss of fusion protein activity on introduction of a *phoP105*::Tn*10*d or *phoP102*::Tn*10*d-Cam allele.

Assays of acid phosphatase, alkaline phosphatase, and β-galactosidase were performed as previously described, Miller et al., 1989, supra and are reported in units as defined in Miller, 1972, Experiments in molecular genetics, p. 352-355, Cold Spring Harbor Laboratory, Cold Spring Harbor, NY, hereby incorporated by reference.

In the mouse virulence and vaccination studies bacteria grown overnight in Luria broth were washed and diluted in normal saline. The wild-type parent strain of CS022 (ATCC 10428) was used for all live vaccine challenge studies. This strain has a 50% lethal dose (LD₅₀) for naive adult BALB/c mice of less than 20 organisms when administered by intraperitoneal (i.p.) injection and 5x10⁴ when administered orally in NaHCO₃. Mice were purchased from Charles River Breeding Laboratories, Inc. (Wilmington, Mass.) and were 5 to 6 weeks of age at initial challenge. All i.p. inoculations were performed as previously described, Miller et al., 1989, supra. Oral challenge experiments were performed with bacteria grown in LB broth and concentrated by centrifugation. The bacteria were resuspended in 0.1 M NaHCO₃ to neutralize stomach acid, and administered as a 0.5-ml bolus to animals under ether anesthesia. Colony counts were performed to accurately access the number of organisms administered. All challenge experiments were performed 1 month after i.p. inoculation and 6 weeks after oral challenge. Challenge inocula were administered by the same route as vaccinations. The care of all animals was under institutional guidelines as set by the animal care committees at the Massachusetts General Hospital and Harvard Medical School.

Protein electrophoresis was performed as follows. One-dimensional protein gel electrophoresis was performed by the method of Laemmli, 1970, Nature 227:680, hereby incorporated by reference, on whole-cell protein extracts of stationary-phase cells grown overnight in Luria broth. The gels were fixed and stained with Coomassie brilliant blue R250 in 10% acetic acid-10% methanol. Two-dimensional protein gel electrophoresis was performed by method of O'Farrell, 1975, J. Biol. Chem. 250:4007, hereby incorporated by reference, on the same whole-cell extracts. Isoelectric focusing using 1.5% pH 3.5 to 10 ampholines (LKB Instruments, Baltimore, Md.) was carried out for 9,600 V h (700 V for 13 h 45 min). The final tube gel pH gradient extended from pH 4.1 to pH 8.1 as measured by a surface pH electrode (BioRad Laboratories, Richmond, Calif.) and colored acetylated cytochrome pI markers (Calbiochem-Behring, La Jolla, Calif.) run in an adjacent tube. The slab gels were silver stained, Merril et al., 1984, Methods Enzymol. 104:441, hereby incorporated by reference.

In the macrophage survival assays experiments were performed as previously described, Miller et al., 1989, supra, by the method of Buchmeier et al., 1989, Infect. Immun. 57:1, hereby incorporated by reference, as modified from the method of Lissner et al, 1983, J. Immunol. 131:3006, hereby incorporated by reference. Stationary-phase cells were opsonized for 30 min in normal mouse serum before exposure to the cultured bone marrow-derived macrophages harvested from BALB/c mice. One hour after infection, gentamicin sulfate (8 µg/ml) was added to kill extracellular bacteria. All time points were done in triplicate and repeated on three separate occasions.

### PhoP^{c} Mutant Strains Are More Effective as Live Vaccines

PhoP^{c} mutant *S. typhimurium* are very effective when used as a live vaccine against mouse typhoid fever and are superior to PhoP⁻ bacteria. As few a 15 PhoP^{c} bacteria protect mice against 10⁵ LD₅₀ (lethal doses 50%) of wild type organisms by the intraperitoneal route (Table 3). This suggests that *pag* gene products are important antigens for protective immunity against mouse typhoid. Preliminary results have documented that antigens recognized by serum of chronic typhoid carriers recognizes some *phoP*-regulated gene products of *S. typhi*. If protective antigens are only expressed within the host, then dead vaccines only grown in rich media may not induce an immune response against these proteins.

The use of different *S. typhimurium* dead vaccine preparations containing different mutations in the *phoP* regulon was evaluated. As can be seen in Table 3 no dead cell preparations (even those containing mixtures of PhoP⁻ and PhoP^{c} bacteria) are as effective vaccines as are live bacteria. This suggests that there are other properties of live vaccines that increase immunogenicity or that important non-PhoP-regulated antigens are not in these preparations. The only protection observed in any animals studied was at the lowest challenge dose for those immunized with PhoP^{c} bacteria. This further suggests that *phoP* activated genes are important protective antigens.

**Table 3**

| *Salmonella* with *phoP* regulon mutations used as a dead vaccine | | | |
|---|---|---|---|
| Strain | Vaccination phenotype | Challenge dose of wild type organisms | |
| | | 6 x 10³ | 6 x 10⁵ |
| None | | (3) | (5) |
| ATCC10428 | wild-type | (8) | (9) |
| CS015 | PhoP⁻ | (10) | (13) |
| CS022 | PhoP^{c} | 2/7(*) | (14) |
| CS022/CS015 | PhoP⁻/PhoP^{c} | (8) | (13) |
| CS015 = *phoP102*::Tn*10d*-*Cam* CS022 = *pho-24* BALB/c mice were immunized twice, 7 days apart, with 5x10⁸ formalin-killed bacteria. Three weeks after the second vaccination, mice were challenged with wild-type organisms at the two doses indicated. The numbers in parentheses indicate no survivors after challenge and mean number of days until death | | | |

| | | | |
|---|---|---|---|
| (*) Ratio of survivors to number challenged. *phoP*^{*c*} indicates the constitutive unregulated expression of *phoP*-activated genes and lack of expression of *phoP* repressed genes. *phoP*^{*-*} indicates a lack of expression of *phoP*-activated genes and expression of *phoP* repressed genes. | | | |

### aroA PhoP Regulon Double Mutant Strains

Recent efforts by Stocker, Levine, and colleagues have focused on the use of strains with auxotrophic mutations in *aro*matic amino acid and purine pathways as live vaccines, Hoseith et al., 1981, Nature 291:238, hereby incorporated by reference, Stocker, 1988, Vaccine 6:141, hereby incorporated by reference, and Levine et al., 1987, J. Clin. Invest. 79:888, hereby incorporated by reference. Purine mutations were found to be too attenuating for immunogenicity, likely because purines are not available to the organism within the mammalian host, Sigwart et al., 1989, Infect. Immun. 57:1858, hereby incorporated by reference. Because auxotrophic mutations may be complemented by homologous recombination events with wild type copies donated from environmental organisms or by acquiring the needed metabolite within the host, it would seem prudent for live vaccines to contain a second attenuating mutation in a different virulence mechanism, (i.e., not just a second mutation in the same metabolic pathway). Additionally, in mice the *aro*A mutants have some residual virulence. Various strains with *aro*A mutations combined with *phoP* regulon mutations were investigated for virulence attenuation and immunogenicity. Table 4 demonstrates that a PhoP⁻ or PhoP^{c} mutation further attenuates *aro*A mutant *S. typhimurium* by at least 100-fold and that, at least at high levels of vaccinating organisms, immunogenicity is retained. Strains with both a *pag*C⁻ and *phoP*^{c} phenotype are also further attenuated than either mutation alone. Therefore, *phoP* regulon mutations may increase the safety or *aroA* live vaccine preparations. It should be noted that strains only with the *aroA* mutation and not also with a mutation in the *prg* or *pag* genes are not within the scope of the invention.

**Table 4A**

| Additional attenuation of *aro*A mutants by PhoP regulon mutations | | | | | | |
|---|---|---|---|---|---|---|
| | | Survivors of varying numbers of *Salmonella* mutant organisms (*) | | | | |
| Strain | Phenotype | 10⁶ | 10⁷ | 10⁸ | 10⁹ | 10¹⁰(**) |
| CS004 | *aro*A- | 6/6 | 1/6 | 0/6 | 0/6 | 6/6 |
| SL3261 | *aro*Adel His⁻ | 6/6 | 1/6 | 0/6 | 0/6 | 6/6 |
| CS322 | *aro*A- PhoP^{c} | 6/6 | 6/6 | 6/6 | 1/6 | 6/6 |
| CS323 | Sl3261 PhoP^{c} | 6/6 | 6/6 | 6/6 | 2/6 | 6/6 |
| CS315 | *aro*A- PhoP⁻ | 6/6 | 6/6 | 6/6 | 2/6 | 6/6 |
| CS316 | SL3261 PhoP⁻ | 6/6 | 6/6 | 6/6 | 1/6 | 6/6 |
| CS026 | *pag*C⁻ PhoP^{c} | 6/6 | 4/6 | 0/6 | 0/6 | 6/6 |

| | | | | | | |
|---|---|---|---|---|---|---|
| (*) Ratio of survivors to number of mice challenged. | | | | | | |
| (**) Indicates oral inoculation all other experiments were intraperitoneal inoculation. CS004 = *aro*A554::rn*10*. SL3261 = *aro*ADEL407 *hisG46*. CS322 = *aro*A554::Tn10 pho-24. CS323 = *aro*ADEL407 pho-24. CS315 = *aro*A554::Tn*10 phoP102*::Tn*10d*-*Cam*. CS316 = *aro*ADEL407 hisG46 phoP102::Tn*10d-Cam*. CS026 = *pag*C1::Tn*phoA pho-24 phoN2 zxx*::6251TN*10d*-*Cam*. | | | | | | |

**Table 4B**

| Protective efficacy of *Salmonella* with *aro*A/*phoP* regulon mutations | | | | |
|---|---|---|---|---|
| | | | Survivors of challenge doses of wild type organisms (*) | |
| Strain | Phenotype | Inoculum | 5 x 10⁵ | 5 x 10⁷ |
| CS004 | *aro*A⁻ | 10⁶ | 4/4 | 5/5 |
| SL3261 | *aro*Adel His⁻ | 10⁶ | 4/4 | 4/5 |
| CS322 | *aro*A⁻ PhoP^{c} | 10⁶ | 5/5 | |
| CS323 | SL3261 PhoP^{c} | 10⁶ | 5/5 | |
| CS322 | *aro*A⁻ PhoP^{c} | 10⁷ | 5/5 | |
| CS323 | SL3261 PhoP^{c} | 10⁷ | 5/5 | |
| CS322 | *aro*A⁻ PhoP^{c} | 10⁸ | | 5/5 |
| CS323 | SL3261 PhoP^{c} | 10⁸ | | 5/5 |
| CS315 | *aro*A⁻ PhoP⁻ | | 5/5 | |
| CS316 | SI3261 PhoP⁻ | 10⁸ | 5/5 | |

| | | | | |
|---|---|---|---|---|
| (*) Ratio of survivors to number of mice challenged. | | | | |
| (**) Indicates oral inoculation all other experiments were intraperitoneal inoculation. CS004 = *aro*A554::rn*10*. SL3261 = *aro*ADEL407 *hisG46*. CS322 = *aro*A554::Tn10 pho-24. CS323 = *aro*ADEL407 pho-24. CS315 = *aro*A554::Tn*10 phoP102*::Tn*10d*-*Cam*. CS316 = *aro*ADEL407 hisG46 phoP102::Tn*10d-Cam*. CS026 = *pag*C1::Tn*phoA pho-24 phoN2 zxx*::6251TN*10d*-*Cam*. | | | | |

### Salmonella typhi phoP Regulon Mutations

The *phoP* regulon is at least partially conserved in *S. typhi* DNA hybridization studies as well as P22 bacteriophage transductional crosses have documented that the *phoP*, *phoQ*, and *pag*C genes appear highly conserved between *S. typhi* and S. typhimurium mutations in these genes in *S. typhi* have been made.

### Salmonella Live Vaccines as Delivery Systems for Heterologous Antigens

The vector used in the vaccine delivery system is a derivative of pJM703.1 described in Miller et al., 1988, J. Bact. 170:2575, hereby incorporated by reference. This vector is an R6K derivative with a deletion in the *pir* gene. R6K derivatives require the protein product of the *pir* gene to replicate. *E. coli* that contain the *pir* gene present as a lambda bacteriophage prophage can support the replication of this vector. Cells that do not contain the *pir* gene will not support the replication of the vector as a plasmid. This vector also contains the *mob* region of RP4 which will allow mobilization into other gram negative bacteria by mating from *E. coli* strains such as SM10lambda *pir*, which can provide the mobilization function in trans.

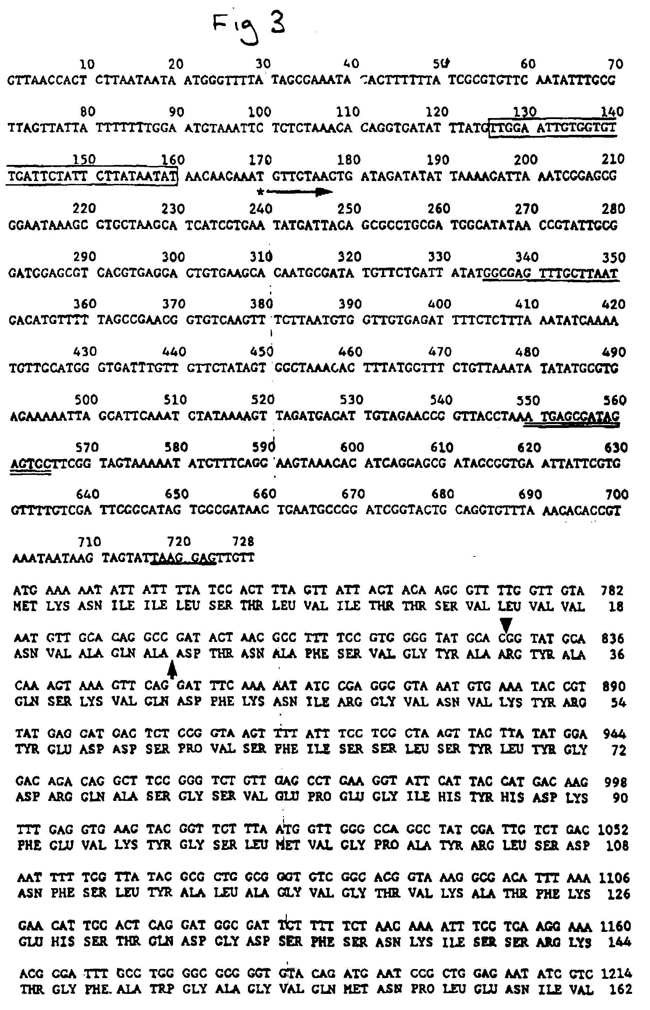

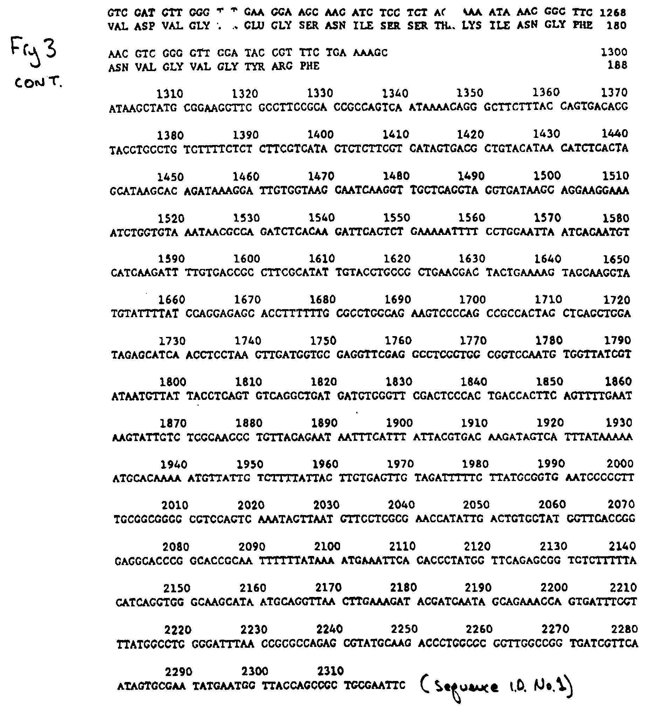

The *pag*C region is shown in Figs. 2 and 3. Fig. 2 shows the restriction endonuclease sites of the *pag*C locus. The heavy bar indicates *pag*C coding sequence. The Tn*phoA* insertion is indicated by a inverted triangle. The direction of transcription is indicated by the arrow and is left to right. The numbers indicate the location of endonuclease sites, in number of base pairs, relative to the start codon of predicted *pag*C translation with positive numbers indicating location downstream of the start codon and negative numbers indicating location upstream of the start codon. A is *Acc*I, B is *Bgl*I, C is *Cla*I, D is *Dra*I, E is *Eco*RI, H is *Hpa*I, N is *Nru*I, P is *Pst*I, S is *Ssp*I, T is *Stu*I, U is *Pvu*II, V is *Eco*RV, and II is *Bg1*II. Fig. 3 shows the DNA sequence (Sequence I.D. No. 1) and translation of *pag*C::Tn*phoA*. The heavy underlined sequence indicates a potential ribosomal binding site. The single and double light underlines indicate sequences in which primers were constructed complementary to these nucleotides for primer extension of RNA analysis. The asterix indicates the approximate start of transcription. The arrow indicates the direction of transcription. The boxed sequences indicate a region that may function in polymerase binding and recognition. The inverted triangle is the site of the sequenced TnphoA insertion junction. The arrow indicates a potential site for single sequence cleavage.

3 kilobases of DNA containing the *pag*C gene (from the *PstI* restriction endonuclease site 1500 nucleotides 5' to the start of *pag*C translation to the EcoRI restriction endonuclease site 1585 nucleotides downstream of *pag*C translation termination) were inserted into the pJM703.1 derivative discussed above. The *pag*C sequence from the *Cla*I restriction endonuclease site was deleted (490 nucleotides) and replaced with a synthetic oligonucleotide polylinker that creates unique restriction endonuclease sites. DNA encoding one or more heterologous proteins, e.g., an antigen, can be inserted into this site. This creates a vector which allows the insertion of multiple foreign genes into the DNA surrounding *pag*C.

The vector can be mobilized into *Salmonella* by mating or any other delivery system, e.g., heat shock, bacteriophage transduction or electroporation. Since it can not replicate, the vector can only insert into *Salmonella* by site specific recombination with the homologous DNA on both sides of the *pag*C gene. This will disrupt and inactivate the native *pag*C locus and replace it with the disrupted *pag*C DNA carried on the vector.

Such recombination events can be identified by marker exchange and selective media if the foreign DNA inserted into the *pag*C locus confers a growth advantage. The insertion of antibiotic resistance genes for selection is less desirable as this could allow an increase in antibiotic resistance in the natural population of bacteria. Genes which confer resistance to substances other than antibiotics e.g., to heavy metals or arsenic (for mercury resistance, see Nucifora et al., 1989, J. Bact., 171:4241-4247, hereby incorporated by reference), can be used to identify transformants. Alternatively, selection can be performed using a *Salmonella* recipient strain that carries an auxotrophic mutation in a metabolic pathway and a vector that carries DNA that compliments the auxotrophic mutation. Many *Salmonella* live vaccine prototypes contain mutations in histidine or purine pathways thus complementation of these metabolic auxotrophies can be used to select for integrants. (Purine mutations specifically have been shown to be too attenuated for use in man.) Further proof of marker exchange can be documented by loss of the ampicillin resistance (carried on the plasmid backbone) or by blot hybridization analysis.

A gene useful for selection can be cloned by complementation of a vaccine strain with a metabolic auxotrophy. Specific examples include the cloning of the DNA encoding both *purB* and *phoP* by complementation of a strain deleted for function of both these genes. *Salmonella* gene libraries have been constructed in a pLAFR cosmid vector (Frindberg et al., 1984, Anal. Biochem. 137:266-267, hereby incorporated by reference) by methods known to those skilled in the art. pLAFR cosmids are broad host range plasmids which can be mobilized into *Salmonella* from *E. coli*. An entire bank of such strains can be mobilized into *Salmonella* vaccine strains and selected for complementation of an auxotrophic defect (e.g., in the case of *purB* growth on media without adenine). The DNA able to complement this defect is then identified and can be cloned into the antigen delivery vector.

As discussed above heterologous genes can be inserted into the polylinker that is inserted into the *pag*C sequence of the vector. The heterologous genes can be under the control of any of numerous environmentally regulated promotor systems which can be expressed in the host and shut off in the laboratory. Because the expression of foreign proteins, especially membrane proteins (as are most important antigens), is frequently toxic to the bacterium, the use of environmentally regulated promoters that would be expressed in mammalian tissues at high levels but which could be grown in the laboratory without expression of heterologous antigens would be very desirable. Additionally, high expression of antigens in host tissues may result in increased attenuation of the organism by diverting the metabolic fuel of the organism to the synthesis of heterologous proteins. If foreign antigens are specifically expressed in host phagocytic cells this may increase the immune response to these proteins as these are the cells responsible for processing antigens.

The promoter systems likely to be useful include those nutritionally regulated promoter systems for which it has been demonstrated that a specific nutrient is not available to bacteria in mammalian hosts. Purines, Sigwart et al., 1989, Infect. Immun., 57:1858 and iron, Finklestein et al., 1983, Rev. Infect. Dis. 5:S759, e.g., are not available within the host. Promoters that are iron regulated, such as the aerobactin gene promoter, as well as promoters for biosynthetic genes in purine pathways, are thus excellent candidates for testing as promoters that can be shut down by growth in high concentrations of these nutrients. Other useful environmentally regulated *Salmonella* promoters include promoters for genes which encode proteins which are specifically expressed within macrophages, e.g., the DnaK and GroEL proteins, which are increased by growth at high temperature, as well as some *phoP* activated gene products, Buchmeier et al., 1990, Science 248:730, hereby incorporated by reference. Therefore, promoters such as the *pag*C 5' controlling sequences and the better characterized promoters for heat shock genes, e.g., GroEL and DnaK, will be expected to be activated specifically within the macrophage. The macrophage is the site of antigen processing and the expression of heat shock genes in macrophages and the wide conservation of heat shock genes in nature may explain the immunodominance of these proteins. A consensus heat shock promoter sequence is known and can be used in the vectors (Cowling et al., 1985, Proc. Natl. Acad. Sci. USA 82:2679, hereby incorporated by reference).

The vectors can include an environmentally regulated T7 polymerase amplification system to express heterologous proteins. For example, the T7 polymerase gene (cloned by Stan Tabor and Charles Richardson, See Current Protocols in Molecular Biology ed. Ausubel et al., 1989, (page 3.5.1.2) John Wiley and Sons, hereby incorporated by reference) under control of an iron regulated promoter, can be included on the vectors described above. We have inserted the aerobactin gene promoter of *E. coli* with the sequence CATTTCTCATTGATAATGAGAATCATTATTGACATAATTGTTATTATTTTACG (Sequence ID No. 2), Delorenzo et al. J. Bact. 169:2624, hereby incorporated by reference, in front of the T7 polymerase gene and demonstrated iron regulation of the gene product. This version of the vector will also include one or more heterologous antigens under the control of T7 polymerase promoters. It is well known that RNA can be synthesized from synthetic oligonucleotide T7 promoters and purified T7 in vitro. When the organism encounters low iron T7 polymerase will be synthesized and high expression of genes with T7 promoters will be facilitated.

The *pag*C gene and *pag*C Gene Product Strains, materials, and methods The following strains, materials, and methods were used in the cloning of *pag*C and in the analysis of the gene and its gene product.

Rich media was Luria broth (LB) and minimal media was M9, Davis et al., 1980, supra. The construction of *S. typhimurium* strain CS119 *pag*C1::Tn*phoA phoN2* zxx::6251 Tn*10d*-*Cam* was previously described, Miller et al., 1989, supra. American Type Culture Collection (ATCC) *S. typhimurium* strain 10428 included CS018 which is isogenic to CS119 except for *phoP105*::Tn*10d*, Miller et al., 1989, supra, CS022 *pho*-*24*, Miller et al., 1990, J. Bacteriol. 172:2485-2490, hereby incorporated by reference, and CS015 *phoP102*::Tn10d-cam, Miller et al., 1989, supra. Other wild type strains used for preparation of chromosomal DNA included *S. typhimurium* LT2 (ATCC 15277), *S. typhimurium* Q1 and *S. drypool* (Dr. J. Peterson U. Texas Medical Branch, Galveston), and *Salmonella typhi* Ty2 (Dr. C*aro*line Hardegree, Food and Drug Administration). pLAFR cosmids were mobilized from *E. coli* to *S. typhimurium* using the *E. coli* strain MM294 containing pRK2013, Friedman et al., 1982, Gene 18:289-296, hereby incorporated by reference. Alkaline phosphatase (AP) activity was screened on solid media using the chromogenic phosphatase substrate 5-bromo-4-chloro-3-indolyl phosphate (XP). AP assays were performed as previously described, Brickman et al., 1975, J. Mol. Biol. 96:307-316, hereby incorporated by reference, and are reported in units as defined by Miller, Miller, 1972, supra, pp. 352-355.

One dimensional protein gel electrophoresis was performed by the method of Laemmli, 1970, Nature, 227:680-685, hereby incorporated by reference, and blot hybridization using antibody to AP was performed as previously described, Peterson et al., 1988, Infect. Immun. 56:2822-2829, hereby incorporated by reference. Whole cell protein extracts were prepared, from saturated cultures grown in LB at 37°C with aeration, by boiling the cells in SDS-*pag*E sample buffer, Laemmli, 1970, supra. Two dimensional gel electrophoresis was performed by the method of O'Farrell, 1975, J. Biol. Chem. 250:4007, hereby incorporated by reference. Proteins in the 10% polyacrylamide slab gels were visualized by silver staining, Merril et al., 1984, Methods in Enzymology, 104:441, hereby incorporated by reference.

Chromosomal DNA was prepared by the method of Mekalanos, 1983, Cell, 35:253-263, hereby incorporated by reference. DNA, size fractionated in ag*aro*se gels, was transferred to nitrocellulose (for blot hybridization) by the method of Southern, 1975, J. Mol. Biol. 98:503-517, hereby incorporated by reference. DNA probes for Southern hybridization analysis were radiolabeled by the random primer method, Frinberg et al., 1984, supra. Plasmid DNA was transformed into *E. coli* and *Salmonella* by calcium chloride and heart shock, Mekalanos, 1983, supra, or by electroporation using a Genepulser apparatus (Biorad, Richmond, Ca.) as recommended by the manufacturer, Dower et al., 1988, Nucl. Acids Res. 16:6127-6145, hereby incorporated by reference. DNA sequencing was performed by the dideoxy chain termination method of Sanger et al., 1977, Proc. Natl. Acad. Sci. USA, 74:5463-5467, hereby incorporated by reference, as modified for use with Sequenase (U.S. Biochemical, Cleveland, Ohio). Oligonucleotides were synthesized on an Applied Biosystems Machine and used as primers for sequencing reactions and primer extension of RNA. Specific primers unique to the two ends of Tn*phoA*, one of which corresponds to the alkaline phosphatase coding sequence and the other to the right IS50 sequence, were used to sequence the junctions of the transposon insertion.

Construction of a *S. typhimurium* cosmid gene bank in pLAFR3 and screening for clones containing the wild type *pag*C DNA was performed as follows. DNA from *S. typhimurium* strain ATCC 10428 was partially digested using the restriction endonuclease Sau3A and then size selected on 10-40% sucrose density gradient. T4 DNA ligase was used to ligate chromosomal DNA of size 20-30 kilobases into the cosmid vector pLAFR3, a derivative of pLAFR1, Friedman et al., 1982, Gene 18:289-296, hereby incorporated by reference, that was digested with the restriction endonuclease *Bam*HI. Cosmid DNA was packaged and transfected into *E. coli* strain DH5-α using extracts purchased from Stratagene, La Jolla, Ca. Colonies were screened by blot hybridization analysis.

The analysis of proteins produced from cloned DNA by in vitro transcription/translation assays was analyzed as follows. These assays were performed with cell free extracts, (Amersham, Arlington Heights, Illinois), and were performed using conditions as described by the manufacturer. The resultant radiolabeled proteins were analyzed by SDS-*pag*E.

RNA was purified from early log and stationary phase *Salmonella* cultures by the hot phenol method, Case et al., 1988, Gene 72:219-236, hereby incorporated by reference, and run in ag*aro*se-formaldehyde gels for blot hybridization analysis, Thomas, 1980, Proc. Natl. Acad. Sci. USA 77:5201, hereby incorporated by reference. Primer extension analysis of RNA was performed as previously described, Miller et al., 1986, Nuc. Acids. Res. 14:7341-7360, hereby incorporated by reference, using AMV reverse transcriptase (Promega, Madison, Wisconsin) and synthesized oligonucleotide primers complementary to nucleotides 335-350 and 550-565 of the *pag*C locus.

Identification of an 18 kDa protein missing in a *pag*C mutant of *S. typhimurium* *pag*C mutant strain CS119 was analyzed by two dimensional protein electrophoresis to detect protein species that might be absent as a result of the Tn*phoA* insertion. Only a single missing protein species, of approximately 18 kD and pI-8.0, was observed when strains, isogenic except for their transposon insertions, were subjected to this analysis. This 18 kDa species was also missing in similar analysis of *Salmonella* strains with mutations *phoP* and *phoQ*. Though two-dimensional protein gel analysis might not detect subtle changes of protein expression in strain CS119, this suggested that a single major protein species was absent as a result of the *pag*C::Tn*phoA* insertion.

Additional examination of the 2-dimensional gel analysis revealed a new protein species of about 45 kDa that is likely the *pag*C-Ap fusion protein. The *pag*C-AP fusion protein was also analyzed by Western blot analysis using antisera to AP and found to be similar in size to native AP (45 kDa) and not expressed in *PhoP-S*. *typhimurium*.

Cloning of the *pag*C::Tn*phoA* insertion Chromosomal DNA was prepared from *S. typhimurium* strain CS119 and a rough physical map of the restriction endonuclease sites in the region of the *pag*C::Tn*phoA* fusion was determined by using a DNA fragment of Tn*phoA* as a probe in blot hybridization analysis. This work indicated that digestion with the restriction endonuclease *eco*RV yielded a single DNA fragment that included the *pag*C::Tn*phoA* insertion in addition to several kilobases of flanking DNA. Chromosomal DNA from strain CS119 was digested with *Eco*RV (blunt end) and ligated into the bacterial plasmid vector pUC19 (New England Biolabs) that had been digested with the restriction endonuclease *Sma*I (blunt end). This DNA was electroporated into the *E. coli* strain DH5-α (BRL) and colonies were plated onto LB agar containing the antibiotics kanamycin (Tn*phoA* encoded and ampicillin (pUC19 encoded). A single ampicillin and kanamycin resistant clone containing a plasmid designated pSM100 was selected for further study.

A radiolabeled DNA probe from pSM100 was constructed and used in Southern hybridization analysis of strain CS119 and its wild type parent ATCC 10428 to prove that the *pag*C::Tn*phoA* fusion had been cloned. The probe contained sequences immediately adjacent to the transposon at the opposite end of the alkaline phosphatase gene [*Hpa*I endonuclease generated DNA fragment that included 186 bases of the right IS50 of the transposon and 1278 bases of *Salmonella* DNA (Fig. 2). As expected, the pSM100 derived probe hybridized to an 11-12 kb *AccI* endonuclease digested DNA fragment from the strain containing the transposon insertion, CS119. This was approximately 7.7kb (size of Tn*phoA*) larger than the 3.9 kB *AccI* fragment present in the wild type strain that hybridizes to the probe. In addition, a derivative of plasmid pSM100, pSM101 (which did not allow expression of the *pag*C-PhoA gene fusion off the *lac* promoter), was transformed into *phoP*- (strain Cs015) and *phoN*- (strain CS019) *Salmonella* strains and the cloned AP activity was found to be dependent on *phoP* for expression. Therefore we concluded that the cloned DNA contained the *pag*C::Tn*phoA* fusion.

The presence of the *pag*C gene was also demonstrated in other strains of *S. typhimurium*, as well as in *S. typhi*, and *S. drypool*. All *Salmonella* strains examined demonstrated similar strong hybridization to an 8.0 kb *Eco*RV and a 3.9 kb *Acci*I restriction endonuclease fragment suggesting that *pag*C is a virulence gene common to *Salmonella* species.

The *pag*C gene probe from nucleotides -46 (with 1 as the first base of the methionine to 802 (*Pst*I site to the *Bgl*II site) failed to cross hybridize to DNA from *Citrobacter freundil, Shigella flexneri, Shigella sonnei, Shigella dysenterial, Escherichia coli, Vibrio cholerae, Vibrio vulnificus, Yersenia entero colitica,* and *Klibsiella pneumonia*.

Cloning of the wild type *pag*C locus DNA and its complementation of the virulence defect of a *S. typhimurium pag*C mutant The same restriction endonuclease fragment described above was used to screen a cosmid gene bank of wild type strain ATCC 10428. A single clone, designated pWP061, contained 18 kilobases of *S. typhimurium* DNA and hybridized strongly to the *pag*C DNA probe. pWP061 was found to contain *Salmonella* DNA identical to that of pSM100 when analyzed by restriction endonuclease analysis and DNA blot hybridization studies. Probes derived from pWP061 were also used in blot hybridization analysis with DNA from wild type and CS119 *S. typhimurium*. Identical hybridization patterns were observed to those seen with pSM100. pWP061 was also mobilized into strain CS119, a *pag*C mutant strain. The resulting strain had wild type virulence for BALB/c mice (a LD₅₀ less than 20 organisms when administered by IP injection). Therefore the cloned DNA complements the virulence defect of a *pag*C mutant strain.

Since, a wild type cosmid containing *pag*C locus DNA was found to complement the virulence defect of a *pag*C mutant *S. typhimurium* strain, it was concluded that the *pag*C protein is an 188 amino acid (18 kDa) membrane (see below) protein essential for survival within microphages and virulence of *S. typhimurium*.

Physical mapping of restriction endonuclease sites, DNA sequencing, and determination of the *pag*C gene product Restriction endonuclease analysis of plasmid pSM100 and pWP061 was performed to obtain a physical map of the *pag*C locus, and, in the case of PSM100, to determine the direction of transcription (Fig. 2). DNA subclones were generated and the Tn*phoA* fusion junctions were sequenced, as well as the *Salmonella* DNA extending from the *Hpa*I site, 828 nucleotides 5' to the *phoA* fusion junction, to the *Eco*RI site 1032 nucleotides 3' to the Tn*phoA* insertion (Fig. 2 and 3). The correct reading frame of the DNA sequence was deduced from that required to synthesize an active AP gene fusion. The deduced amino acid sequence of this open reading frame was predicted to encode a 188 amino acid protein with a predicted pI+8.2. This data were consistent with the 2-D polyacrylamide gel analysis of strain CS119 in which an 18 kDa protein of approximate pI+8.0 was absent. No other open reading frames, predicted to encode peptides larger than 30 amino acids, were found.

The deduced amino acid sequence of the 188 amino acid open reading frame contains a methionine start codon 33 amino acids from the fusion of *pag*C and AP (Fig. 3). This 33 amino acid *pag*C contribution to the fusion protein was consistent with the size observed in Western blot analysis and contains a hydrophobic N-terminal region, identified by the method of Kyle et al., 1982, J. Mol. Biol. 157:105-132, hereby incorporated by reference, that is a typical bacterial signal sequence, Von Heinje, 1985, J. Mol. Biol. 184:99-105, hereby incorporated by reference. Specifically, amino acid 2 is a positively charged lysine, followed by a hydrophobic domain and amino acid 24 is a negatively charged aspartate residue. A consensus cleavage site for this leader peptide is predicted to be at an alanine residue at amino acid 23, Von Heinje, 1984, J. Mol. Biol. 173:243-251, hereby incorporated by reference. The DNA sequence also revealed a typical ribosomal binding site, Shine et al., 1974, Proc. Natl. Acad. Sci. USA 71:1342-1346, hereby incorporated by reference, at 6-2 nucleotides 5' to the predicted start of translation (Fig. 3) nucleotides 717-723). This suggested that the open reading frame was, in fact, translated and further supported the assumption that this was the deduced amino acid sequence of the *pag*C protein interrupted by the Tn*phoA* insertion (Fig. 3).

In vitro synthesis of proteins by the cloned *pag*C locus To detect if other proteins were encoded by *pag*C and to determine the approximate size of the *pag*C gene product, an *in vitro* coupled transcription/translation analysis was performed. A 5.3 kilobase *Eco*RI fragment of pWP061 was inserted into pUC19 so that the *pag*C gene would not be expressed off the *lac* promotor. This plasmid was used in an *in vitro* coupled transcription-translation assay. A single protein of approximately 22 kilodaltons was synthesized by the cell free system. The size was compatible with this being the precursor of the *pag*C protein containing its leader peptide. These data further support the conclusion the single and the single *pag*C gene product had been identified.

Identification of the *pag*C encoded RNA An approximately 1100 nucleotide RNA is encoded by *pag*C. The *pag*C gene is highly expressed by cells with a *phoP* constitutive phenotype of *pag* activation, as compared to wild type and *phoP* constitutive phenotype of *pag* activation, as compared to wild type and *phoP*- bacteria. In these blot hybridization experiments *pag*C is only detected in wild type cells grown in rich media during stationary growth. This result, coupled with previous work, Miller et al., 1989, supra, Miller et al., 1990, supra, demonstrates that *pag*C is transcriptionally regulated by the *phoP* gene products and is only expressed during early logarithmic phase growth in rich media by cells with a *phoP* constitutive phenotype.

The size of the *pag*C transcript is approximately 500 nucleotides greater than that necessary to encode the 188 amino acid protein. Primer extension analysis of *Salmonella* RNA using oligonucleotide primers specific for *pag*C sequence was performed to determine the approximate start site of transcription and to determine whether these nucleotides might be transcribed 5' or 3' to the 188 amino acid *pag*C gene product. Primer extension analysis with an oligonucleotide predicted to be complementary to nucleotides 550-565 of *pag*C, 150 nucleotides 5' to the predicted start codon, resulted in an approximately 300 nucleotide primer extension product. Therefore a primer further upstream was constructed complementary to nucleotides 335-350 of *pag*C and used in a similar analysis. A primer extension product of 180 nucleotides was observed to be primer specific. This is consistent with transcription starting at nucleotide 170 (Fig. 3). Upstream of the predicted transcriptional start, at nucleotides 153-160, a classic RNA polymerase binding site was observed with the sequence TATAAT at -12 nucleotides as well as the sequence TAATAT at -10 nucleotides. No complete matches were observed for the consensus RNA polymerase recognition site (TTGACA) 15-21 nucleotides upstream from the -10 region. AT -39 (126-131) nucleotides (TTGGAA), -38 (127-132) nucleotides (TTGTGG), and -25 (135-140) nucleotides (TTGATT) are sequences that have matches with the most frequently conserved nucleotides of this sequence.

Based on the above results transcription was predicted to terminate near the translational stop codon of the 188 amino acid protein (nucleotide 1295, Fig. 3). Indeed, a stem loop configuration was found at nucleotides 1309-1330 that may function as a transcription terminator. This was consistent with the lack of evidence of open reading frames downstream of the 188 amino acid protein and the lack of synthesis of other transcription/translation using the cloned *pag*C DNA. This further suggests that the *pag*C::Tn*phoA* insertion inactivated the synthesis of only a single protein.

Similarity of *pag*C to Ail and Lom A computer analysis of protein similarity using the National Biomedical Research Foundation/Protein Identification Resource, George et al., 1986, Nucleic Acids Res. 14:11-15, hereby incorporated by reference, protein sequence base was conducted to identify other proteins that had similarity to *pag*C in an attempt to find clues to the molecular function of this protein. Remarkably, *pag*C was found to be similar to a bacteriophage lambda protein, Lom, that has been localized to the outer membrane in minicell analysis, Court et al., 1983, Lambda II, Hendrix, R.W. et al. ed. Cold Spring Harbor Laboratory (Cold Spring Harbor NY), pp. 251-277, hereby incorporated by reference, and demonstrated to be expressed by lambda lysogens of *E. coli*, Barondess, et al, 1990, Nature 346:871-874, hereby incorporated by reference. Recently, the deduced amino acid sequence of the cloned *ail* gene product of *Y. enterocolitica* was determined and found to also be similar to Lom, Miller et al., 1990b, J. Bacteriol. 172:1062-1069. Therefore, a protein family sequence alignment was performed using a computer algorithm that establishes protein sequence families and consensus sequences, Smith et al., 1990, Proc. Natl. Acad. Sci. 87:118-122, hereby incorporated by reference. The formation of this family is indicated by the internal data base values of similarity between these proteins : *pag*C and Lom (107.8), *pag*C and Ail (104.7), and Ail and Lom (89.8). These same proteins were searched against 314 control sequences in the data base and mean values and ranges were 39.3 (7.3-52.9) *pag*C, 37.4 (7.3-52.9) Ail, and 42.1 (7.0-61.9) Lom. The similarity values for this protein family are all greater than 3.5 standard deviations above the highest score obtained for similarity to the 314 random sequences. No other similarities or other family members were found in the database. Regions of similarity are located not only in the leader peptide transmembrane domains but throughout the protein.

### pagC Mutant Strains Are Attenuated For Virulence

*Salmonella typhimurium* strains with a *pag*C mutation are most likely inactivated for the *phoP*-regulated gene product, as these strains are attenuated for virulence by at least 1,000-fold.

### Attenuation of Bacterial Virulence by Constitutive Expression of Two-component Regulatory Systems.

The virulence of a bacterium can be attenuated by inducing a mutation or which results in the constitutive expression of genes under the control of a two-component regulatory system or by inducing a mutation that inactivates a gene under the control of the two-component systems. A balance between the expression of the genes under the control of the two-component system, e.g., between *pag* and *prg* gene expression, and possibly beteen two-component system regulated genes and other genes, is necessary for full virulence. Mutations that disrupt this balance, e.g., mutations that cause the constitutive expression of a gene under the control of the two-component system, or a mutation that inactivates a gene under the control of the two-component system, e.g., the *pag* gene, reduce virulence.

Constitutive mutations in two-component regulators can be identified by the use of a strain containing a recorder gene fusion to a gene regulated by the two-component system. Such gene fusions would most typically include DNA encoding the *lac*Z gene or alkaline phosphatase fused to a gene under the control of the two-component system. Strains containing fusions that are (as compared to wild type or parental strains) highly expressed in an unregulated fashion, i.e., constitutive, can be detected by increased color on chromogenic substrates for the enzymes. To detect constitutive mutations a cloned virulence regulator could be mutagenized e.g., by passage through an E. coli strain defective in DNA repair or by chemical mutagenesis. The mutated DNA for the regulator would then be transferred to the strain containing the gene fusion and constitutive mutations identified by the high gene fusion expression (blue color in the case of a *lac*Z fusion grown on media containing X-gal). Constitutive mutations in a component of a two-component regulatory system could also be made by in vitro mutagenesis after other constitutive mutations have been sequenced and a specific amino acid change responsible for constitutivity identified. Putting several amino acid changes that all result in a PhoP constitutive phenotype would result in a decreased frequency of reversion by spontaneous base changes. A constitutive mutation could also be constructed by deletion of the portion of the amino terminus of the phospho-accepting regulator which contains the phosphoacceptor domain e.g., deletion of sequences encoding amino acids amino terminal to amino acid 119 in the *pho*P gene or deletion of analogous phospho accepting sequences in genes of other two-component regulatory systems. This could result in a conformational change similar to that induced by phosphorylation and result in increased DNA binding and transcriptional activation.

Our *Salmonella* cells are useful as sources of immunological protection against diseases, e.g., typhoid fever and related diseases, in an animal, e.g., a mammal, e.g., a human, in particular as the basis of a live-cell vaccine capable of colonizing the inoculated animal's intestine and provoking a strong immune reaction. Appropriate dosages and conditions of administration of such a live, attenuated vaccine are as described in Holem et al., Acute Enteric Infections in Children, New Prospects for Treatment and Prevention (1981) Elsevier/North-Holland biomedical Press, Ch. 26, pp. 443 et seq. (Levine et al.), hereby incorporated by reference.

Other embodiments, e.g., strains which in addition to a phoP related mutation or genetic alteration also contain an attenuating mutation in another gene, e.g., an *aro*matic amino acid synthetic gene, e.g., *aro*A or *aro*D, or in cya gene (adenylate cyclase) or crp gene (adenylate cyclase receptor) are contemplated.

## Claims (Claims for the following Contracting State(s): AT, BE, CH, LI, DE, DK, FR, GB, IT, LU, MC, NL, SE)

1. A vaccine comprising a *Salmonella* cell the virulence of which is attenuated by a first mutation in the *phoP* regulatory region causing constitutive expression of a gene under the control of said region and by a second mutation at a *pag* or *prg* gene, neither mutation comprising the insertion of an antibiotic resistance gene.

2. A vaccine comprising a *Salmonella* cell the virulence of which is attenuated by a mutation in a *pag* or a *prg* gene, the mutation not comprising the insertion of an antibiotic resistance gene.

3. A *Salmonella* cell which constitutively expresses a *phoP* regulatory region regulated gene and which comprises a virulence attenuating mutation in a *prg* or a *pag* gene, the virulence attenuating mutation not comprising the insertion of an antibiotic resistance gene.

4. A *Salmonella* cell which comprises a virulence attenuating mutation in a *pag* or a *prg* gene, the virulence attenuating mutation not comprising the insertion of an antibiotic resistance gene.

5. A live *Salmonella* cell into a *pag* or a *prg* gene of which is inserted DNA encoding a heterologous protein or a regulatory element of the gene for the heterologous protein, whereby the virulence of said cell is attenuated by said insertion, the DNA not comprising an antibiotic resistance gene.

6. A live *Salmonella* cell according to Claim 5, wherein said DNA encoding a heterologous protein is under the control of an environmentally regulated promoter.

7. A vector capable of integrating into the chromosome of *Salmonella* comprising a first DNA sequence encoding a heterologous protein, a second DNA sequence encoding a marker, and a third DNA sequence encoding mutationally inactivated *Salmonella* virulence factor, said virulence factor being encoded by a *pag* or *prg* gene which has been mutationally inactivated otherwise than by insertion of an antibiotic resistance gene.

## Claims (Claims for the following Contracting State(s): ES, GR)

1. A vaccine comprising a *Salmonella* cell the virulence of which is attenuated by a first mutation in the *phoP* regulatory region causing constitutive expression of a gene under the control of said region and by a second mutation at a *pag* or *prg* gene, neither mutation comprising the insertion of an antibiotic resistance gene.

2. A vaccine comprising a *Salmonella* cell the virulence of which is attenuated by a mutation in a *pag* or a *prg* gene, the mutation not comprising the insertion of an antibiotic resistance gene.

3. A *Salmonella* cell which constitutively expresses a *phoP* regulatory region regulated gene and which comprises a virulence attenuating mutation in a *prg* or a *pag* gene, the virulence attenuating mutation not comprising the insertion of an antibiotic resistance gene.

4. A *Salmonella* cell which comprises a virulence attenuating mutation in a *pag* or a *prg* gene, the virulence attenuating mutation not comprising the insertion of an antibiotic resistance gene.

5. A method of producing a *Salmonella* cell the virulence of which is attenuated, or a vaccine comprising such a cell, the method comprising introducing a first mutation at the *phoP* regulatory region of a *Salmonella* cell such that a gene under the control of said region is constitutively expressed, and a second virulence attenuating mutation at the *pag* or *prg* gene of that cell, neither mutation comprising the insertion of an antibiotic resistance gene.

6. A method of producing a *Salmonella* cell the virulence of which is attenuated, or a vaccine comprising such a cell, the method comprising introducing a virulence attenuating mutation at the *pag* or *prg* gene of a *Salmonella* cell the virulence of which has been attenuated by a mutation at a *phoP* regulatory region, the *phoP* mutation causing constitutive expression of a gene under the control of said region, neither mutation comprising the insertion of an antibiotic resistance gene.

7. A method of producing a *Salmonella* cell the virulence of which is attenuated, or a vaccine comprising such a cell, the method comprising introducing a mutation at a *pag* or *prg* gene of a *Salmonella* cell, the mutation not comprising the insertion of an antibiotic resistance gene.

8. A method of producing a vaccine, the method comprising bringing a *Salmonella* cell into association with a pharmacologically acceptable carrier or diluent, in which the virulence of the *Salmonella* cell is attenuated by a first mutation in the *phoP* regulatory region causing constitutive expression of a gene under the control of said region and by a second mutation at a *pag* or *prg* gene, neither mutation comprising the insertion of an antibiotic resistance gene.

9. A method of producing a vaccine, the method comprising bringing a *Salmonella* cell into association with a pharmacologically acceptable carrier or diluent, in which the virulence of the *Salmonella* cell is attenuated by a mutation in a *pag* or a *prg* gene, the mutation not comprising the insertion of an antibiotic resistance gene.

10. A vaccine, a cell or a method according to any preceding claim, characterised in that the mutation in the *pag* or *prg* gene comprises the insertion of DNA encoding a heterologous protein or a regulatory element of the gene for the heterologous protein, the DNA not comprising an antibiotic resistance gene.

11. A vaccine, a cell or a method according to Claim 10, characterised in that the DNA encoding the heterologous protein is under the control of an environmentally regulated promoter.

12. A live *Salmonella* cell into a *pag* or a *prg* gene of which is inserted DNA encoding a heterologous protein or a regulatory element of the gene for the heterologous protein, whereby the virulence of said cell is attenuated by said insertion, the DNA not comprising an antibiotic resistance gene.

13. A method of producing a live *Salmonella* cell useful in a vaccine, the method comprising inserting DNA encoding a heterologous protein, or a regulatory element of the gene for the heterologous protein, into a *pag* or a *prg* gene of a *Salmonella* cell so that the virulence of the cell is thereby attenuated, the DNA not comprising an antibiotic resistance gene..

14. A cell according to Claim 12 or a method according to Claim 13, in which said DNA encoding a heterologous protein is under the control of an environmentally regulated promoter.

15. A vector capable of integrating into the chromosome of *Salmonella* comprising a first DNA sequence encoding a heterologous protein, a second DNA sequence encoding a marker, and a third DNA sequence encoding mutationally inactivated *Salmonella* virulence factor, said virulence factor being encoded by a *pag* or *prg* gene which has been mutationally inactivated otherwise than by insertion of an antibiotic resistance gene.

16. A method of producing a vector capable of integrating into the chromosome of *Salmonella*, the method comprising bringing into conjunction (a) a first DNA sequence encoding a heterologous protein; (b) a second DNA sequence encoding a marker; and (c) and a third DNA sequence encoding mutationally inactivated *Salmonella* virulence factor, said virulence factor being encoded by a *pag* or *prg* gene which has been mutationally inactivated otherwise than by insertion of an antibiotic resistance gene.

## Patentansprüche (Patentansprüche für folgende(n) Vertragsstaat(en): AT, BE, CH, LI, DE, DK, FR, GB, IT, LU, MC, NL, SE)

1. Impfstoff, umfassend eine *Salmonellen*-Zelle, deren Virulenz abgeschwächt wird durch eine erste Mutation in der *phoP*-Regulationsregion, so daß eine konstitutive Ausprägung eines Gens unter der Kontrolle der genannten Region herbeigeführt wird, und durch eine zweite Mutation an einem *pag*- oder *prg*-Gen, wobei keine der Mutationen das Einfügen eines Antibiotikaresistenzgens umfaßt.

2. Impfstoff, umfassend eine *Salmonellen*-Zelle, deren Virulenz abgeschwächt wird durch eine Mutation in einem *pag*- oder *prg*-Gen, wobei die Mutation nicht das Einfügen eines Antibiotikaresistenzgens umfaßt.

3. *Salmonellen*-Zelle, die konstitutiv ein *phoP*-regulationsregionreguliertes Gen ausprägt und die eine virulenzabschwächende Mutation in einem *prg*- oder *pag*-Gen umfaßt, wobei die virulenzabschwächende Mutation nicht das Einfügen eines Antibiotikaresistenzgens umfaßt.

4. *Salmonellen*-Zelle, die eine virulenzabschwächende Mutation in einem *pag*- oder *prg*-Gen umfaßt, wobei die virulenzabschwächende Mutation nicht das Einfügen eines Antibiotikaresistenzgens umfaßt.

5. Lebende *Salmonellen*-Zelle, in deren *pag-* oder *prg*-Gen DNA eingefügt wird, die ein heterologes Protein oder ein Regulationselement des Gens für das heterologe Protein kodiert, so daß die Virulenz der genannten Zelle durch das genannte Einfügen abgeschwächt wird, wobei die DNA kein Antibiotikaresistenzgen umfaßt.

6. Lebende *Salmonellen*-Zelle nach Anspruch 5, bei der sich die genannte DNA, die ein heterologes Protein kodiert, unter der Kontrolle eines umgebungsregulierten Promoters befindet.

7. Vektor, der in der Lage ist, sich in das Chromosom von *Salmonellen* zu integrieren, umfassend eine erste DNA-Sequenz, die ein heterologes Protein kodiert, eine zweite DNA-Sequenz, die einen Marker kodiert, und eine dritte DNA-Sequenz, die einen mutationsinaktivierten *Salmonellen*-Virulenzfaktor kodiert, wobei der genannte Virulenzfaktor durch ein *pag*- oder *prg*-Gen kodiert wird, das auf andere Weise als durch Einfügen eines Antibiotikaresistenzgens mutationsinaktiviert wurde.

## Patentansprüche (Patentansprüche für folgende(n) Vertragsstaat(en): ES, GR)

1. Impfstoff, umfassend eine *Salmonellen*-Zelle, deren Virulenz abgeschwächt wird durch eine erste Mutation in der *pho*P-Regulationsregion, so daß eine konstitutive Ausprägung eines Gens unter der Kontrolle der genannten Region herbeigeführt wird, und durch eine zweite Mutation an einem *pag*- oder *prg*-Gen, wobei keine der Mutationen das Einfügen eines Antibiotikaresistenzgens umfaßt.

2. Impfstoff, umfassend eine *Salmonellen*-Zelle, deren Virulenz abgeschwächt wird durch eine Mutation in einem *pag*- oder *prg*-Gen, wobei die Mutation nicht das Einfügen eines Antibiotikaresistenzgens umfaßt.

3. *Salmonellen*-Zelle, die konstitutiv ein *phoP*-regulationsregionreguliertes Gen ausprägt und die eine virulenzabschwächende Mutation in einem *prg*- oder *pag*-Gen umfaßt, wobei die virulenzabschwächende Mutation nicht das Einfügen eines Antibiotikaresistenzgens umfaßt.

4. *Salmonellen*-Zelle, die eine virulenzabschwächende Mutation in einem *pag*- oder *prg*-Gen umfaßt, wobei die virulenzabschwächende Mutation nicht das Einfügen eines Antibiotikaresistenzgens umfaßt.

5. Verfahren zum Herstellen einer *Salmonellen*-Zelle, deren Virulenz abgeschwächt wird, oder eines Impfstoffes, der eine solche Zelle umfaßt, wobei das Verfahren die folgenden Schritte umfaßt: Einleiten einer ersten Mutation an der *phoP*-Regulationsregion einer *Salmonellen*-Zelle, so daß ein Gen unter der Kontrolle der genannten Region konstitutiv ausgeprägt wird, und einer zweiten virulenzabschwächenden Mutation am *pag*- oder *prg*-Gen dieser Zelle, wobei keine der Mutationen das Einfügen eines Antibiotikaresistenzgens umfaßt.

6. Verfahren zum Herstellen einer *Salmonellen*-Zelle, deren Virulenz abgeschwächt wird, oder eines Impfstoffes, der eine solche Zelle umfaßt, wobei das Verfahren die folgenden Schritte umfaßt: Einleiten einer virulenzabschwächenden Mutation am *pag*- oder *prg*-Gen einer *Salmonellen*-Zelle, deren Virulenz durch eine Mutation an einer *phoP*-Regulationsregion abgeschwächt wurde, wobei die *phoP*-Mutation eine konstitutive Ausprägung eines Gens unter der Kontrolle der genannten Region bewirkt, wobei keine der Mutationen das Einfügen eines Antibiotikaresistenzgens umfaßt.

7. Verfahren zum Herstellen einer *Salmonellen*-Zelle, deren Virulenz abgeschwächt wird, oder eines Impfstoffes, der eine solche Zelle umfaßt, wobei das Verfahren die folgenden Schritte umfaßt: Einleiten einer Mutation an einem *pag*- oder *prg*-Gen einer *Salmonellen*-Zelle, wobei die Mutation nicht das Einfügen eines Antibiotikaresistenzgens umfaßt.

8. Verfahren zum Herstellen eines Impfstoffes, wobei das Verfahren die folgenden Schritte umfaßt: Bringen einer *Salmonellen*-Zelle in Assoziation mit einem pharmakologisch akzeptablen Träger oder Verdünnungsmittel, wobei die Virulenz der *Salmonellen*-Zelle abgeschwächt wird durch eine erste Mutation in der *phoP*-Regulationsregion, was eine konstitutive Ausprägung eines Gens unter der Kontrolle der genannten Region bewirkt, und durch eine zweite Mutation an einem *pag*- oder *prg*-Gen, wobei keine der Mutationen das Einfügen eines Antibiotikaresistenzgens umfaßt.

9. Verfahren zum Herstellen eines Impfstoffes, wobei das Verfahren die folgenden Schritte umfaßt: Bringen einer *Salmonellen*-Zelle in Assoziation mit einem pharmakologisch akzeptablen Träger oder Verdünnungsmittel, wobei die Virulenz der *Salmonellen*-Zelle abgeschwächt wird durch eine Mutation in einem *pag*- oder *prg*-Gen, wobei die Mutation nicht das Einfügen eines Antibiotikaresistenzgens umfaßt.

10. Impfstoff, Zelle oder Verfahren nach einem der vorherigen Ansprüche, dadurch gekennzeichnet, daß die Mutation in dem *pag*- oder *prg*-Gen das Einfügen von DNA umfaßt, die ein heterologes Protein oder ein Regulationselement des Gens für das heterologe Protein kodiert, wobei die DNA kein Antibiotikaresistenzgen umfaßt.

11. Impfstoff, Zelle oder Verfahren nach Anspruch 10, dadurch gekennzeichnet, daß sich die DNA, die das heterologe Protein kodiert, unter der Kontrolle eines umgebungsregulierten Promoters befindet.

12. Lebende *Salmonellen*-Zelle, in deren *pag*- oder *prg*-Gen DNA eingefügt wird, die ein heterologes Protein oder ein Regulationselement des Gens für das heterologe Protein kodiert, so daß die Virulenz der genannten Zelle durch das genannte Einfügen abgeschwächt wird, wobei die DNA kein Antibiotikaresistenzgen umfaßt.

13. Verfahren zum Herstellen einer lebenden *Salmonellen*-Zelle, die in einem Impfstoff nützlich ist, wobei das Verfahren das Einfügen von DNA, die ein heterologes Protein oder ein Regulationselement des Gens für das heterologe Protein kodiert, in ein *pag*- oder *prg*-Gen einer *Salmonellen*-Zelle umfaßt, so daß die Virulenz der Zelle dadurch abgeschwächt wird, wobei die DNA kein Antibiotikaresistenzgenz umfaßt.

14. Zelle nach Anspruch 12 oder Verfahren nach Anspruch 13, bei der/dem sich die genannte DNA, die ein heterologes Protein kodiert, unter der Kontrolle eines umgebungsregulierten Promoters befindet.

15. Vektor, der in der Lage ist, sich in das Chromosom von *Salmonellen* zu integrieren, umfassend eine erste DNA-Sequenz, die ein heterologes Protein kodiert, eine zweite DNA-Sequenz, die einen Marker kodiert, und eine dritte DNA-Sequenz, die einen mutationsinaktivierten *Salmonellen*-Virulenzfaktor kodiert, wobei der genannte Virulenzfaktor durch ein *pag*- oder *prg-*Gen kodiert wird, das auf andere Weise als durch Einfügen eines Antibiotikaresistenzgens mutationsinaktiviert wurde.

16. Verfahren zum Herstellen eines Vektors, der in der Lage ist, sich in das Chromosom von *Salmonellen* zu integrieren, wobei das Verfahren folgendes umfaßt: Verbinden (a) einer ersten DNA-Sequenz, die ein heterologes Protein kodiert; (b) einer zweiten DNA-Sequenz, die einen Marker kodiert; und (c) einer dritten DNA-Sequenz, die einen mutationsinaktivierten *Salmonellen*-Virulenzfaktor kodiert, wobei der genannte Virulenzfaktor durch ein *pag*- oder *prg*-Gen kodiert wird, das auf andere Weise als durch Einfügen eines Antibiotikaresistenzgens mutationsinaktiviert wurde.

## Revendications (Revendications pour l'(les) Etat(s) contractant(s) suivant(s): AT, BE, CH, LI, DE, DK, FR, GB, IT, LU, MC, NL, SE)

1. Vaccin comprenant une cellule *Salmonella* dont la virulence est atténuée par une première mutation dans la région de régulation *phoP* causant une expression constitutive d'un gène sous le contrôle de ladite région et par une deuxième mutation à un gène *pag* ou *prg*, aucune desdites mutations ne comprenant l'insertion d'un gène d'antibiorésistance.

2. Vaccin comprenant une cellule *Salmonella* dont la virulence est atténuée par une mutation dans un gène *pag* ou *prg*, la mutation ne comprenant pas l'insertion d'un gène d'antibiorésistance.

3. Cellule *Salmonella* qui exprime constitutivement un gêne régulé par la région de régulation *phoP* et qui comprend une mutation d'atténuation de virulence dans un gène *prg* ou *pag*, la mutation d'atténuation de virulence ne comprenant pas l'insertion d'un gène d'antibiorésistance.

4. Cellule *Salmonella* qui comprend une mutation d'atténuation de virulence dans un gène *pag* ou *prg*, la mutation d'atténuation de virulence ne comprenant pas l'insertion d'un gène d'antibiorésistance.

5. Cellule *Salmonella* vivante dans un gène *pag* ou *prg* de laquelle est inséré un ADN codant une protéine hétérologue ou un élément régulateur du gène pour la protéine hétérologue, grâce à quoi la virulence de ladite cellule est atténuée par ladite insertion, l'ADN ne comprenant pas un gène d'antibiorésistance.

6. Cellule *Salmonella* vivante selon la Revendication 5, dans laquelle ledit ADN codant une protéine hétérologue est sous le contrôle d'un promoteur à régulation environnementale.

7. Vecteur capable d'intégrer dans le chromosome de *Salmonella* comprenant une première séquence d'ADN codant une protéine hétérologue une deuxième séquence d'ADN codant un marqueur antigénique, et une troisième séquence d'ADN codant un facteur de virulence de *Salmonella* inactivé par mutation, ledit facteur de virulence étant codé par un gène *pag* ou *prg* qui a été inactivé par mutation autrement que par insertion d'un gène d'antibiorésistance.

## Revendications (Revendications pour l'(les) Etat(s) contractant(s) suivant(s): ES, GR)

1. Vaccin comprenant une cellule *Salmonella* dont la virulence est atténuée par une première mutation dans la région de régulation *phoP* causant une expression constitutive d'un gène sous le contrôle de ladite région et par une deuxième mutation à un gène *pag* ou *prg*, aucune desdites mutations ne comprenant l'insertion d'un gène d'antibiorésistance.

2. Vaccin comprenant une cellule *Salmonella* dont la virulence est atténuée par une mutation dans un gène *pag* ou *prg*, la mutation ne comprenant pas l'insertion d'un gène d'antibiorésistance.

3. Cellule *Salmonella* qui exprime constitutivement un gène régulé par la région de régulation *phoP* et qui comprend une mutation d'atténuation de virulence dans un gène *prg* ou *pag*, la mutation d'atténuation de virulence ne comprenant pas l'insertion d'un gène d'antibiorésistance.

4. Cellule *Salmonella* qui comprend une mutation d'atténuation de virulence dans un gène *pag* ou *prg*, la mutation d'atténuation de virulence ne comprenant pas l'insertion d'un gène d'antibiorésistance.

5. Méthode de production d'une cellule *Salmonella* dont la virulence est atténuée, ou d'un vaccin comprenant une telle cellule, la méthode comprenant l'introduction d'une première mutation à la région de régulation *phoP* d'une cellule *Salmonella* telle qu'un gène sous le contrôle de ladite région est exprimé constitutivement, et d'une deuxième mutation d'atténuation de virulence au gène *pag* ou *prg* de cette cellule, aucune des deux mutations ne comprenant l'insertion d'un gène d'antibiorésistance.

6. Méthode de production d'une cellule *Salmonella* dont la virulence est atténuée, ou d'un vaccin comprenant une telle cellule, la méthode comprenant l'introduction d'une mutation d'atténuation de virulence au gène *pag* ou *prg* d'une cellule *Salmonella* dont la virulence a été atténuée par une mutation à une région de régulation *phoP*, la mutation *phoP* causant l'expression constitutive d'un gène sous le contrôle de ladite région, aucune mutation ne comprenant l'insertion d'un gène d'antibiorésistance.

7. Méthode de production d'une cellule *Salmonella* dont la virulence est atténuée, ou d'un vaccin comprenant une telle cellule, la méthode comprenant l'introduction d'une mutation à un gène *pag* ou *prg* d'une cellule *Salmonella*, la mutation ne comprenant pas l'insertion d'un gène d'antibiorésistance.

8. Méthode de production d'un vaccin, la méthode comprenant la mise en association d'une cellule *Salmonella* avec un porteur ou diluant acceptable pharmacologiquement, dans laquelle la virulence de la cellule *Salmonella* est atténuée par une première mutation dans la région de régulation *phoP* causant une expression constitutive d'un gène sous le contrôle de ladite région et par une deuxième mutation à un gène *pag* ou *prg*, aucune des deux mutations ne comprenant l'insertion d'un gène d'antibiorésistance.

9. Méthode de production d'un vaccin, la méthode comprenant la mise en association d'une cellule *Salmonella* avec un porteur ou un diluant acceptable pharmacologiquement, dans laquelle la virulence de la cellule *Salmonella* est atténuée par une mutation dans un gène *pag* ou *prg*, la mutation ne comprenant pas l'insertion d'un gène d'antibiorésistance.

10. Vaccin, cellule ou méthode selon l'une quelconque des revendications précédentes, caractérisé en ce que la mutation dans le gène *pag* ou *prg* comprend l'insertion d'ADN codant une protéine hétérologue ou un élément de régulation du gène pour la protéine hétérologue, l'ADN ne comprenant pas un gène d'antibiorésistance.

11. Vaccin, cellule ou méthode selon la Revendication 10, caractérisé en ce que l'ADN codant la protéine hétérologue est sous le contrôle d'un promoteur à régulation environnementale.

12. Cellule *Salmonella* vivante dans un gène *pag* ou *prg* de laquelle est inséré un ADN codant une protéine hétérologue ou un élément régulateur du gène pour la protéine hétérologue, grâce à quoi la virulence de ladite cellule est atténuée par ladite insertion, l'ADN ne comprenant pas un gène d'antibiorésistance.

13. Méthode de production d'une cellule *Salmonella* vivante utile dans un vaccin, la méthode comprenant l'insertion d'ADN codant une protéine hétérologue, ou un élément de régulation du gène pour la protéine hétérologue, dans un gène *pag* ou *prg* d'une cellule *Salmonella* de telle sorte que la virulence de la cellule soit ainsi atténuée, l'ADN ne comprenant pas un gène d'antibiorésistance.

14. Cellule *Salmonella* selon la Revendication 12, ou méthode selon la Revendication 13, dans laquelle ledit ADN codant une protéine hétérologue est sous le contrôle d'un promoteur à régulation environnementale.

15. Vecteur capable de s'intégrer dans le chromosome de *Salmonella* comprenant une première séquence d'ADN codant une protéine hétérologue, une deuxième séquence d'ADN codant un marqueur, et une troisième séquence d'ADN codant un facteur de virulence de *Salmonella* inactivé par mutation, ledit facteur de virulence étant codé par un gène *pag* ou *prg* qui a été inactivé par mutation autrement que par insertion d'un gène d'antibiorésistance.

16. Méthode de production d'un vecteur capable de s'intégrer dans le chromosome de *Salmonella*, la méthode comprenant la mise en conjonction (a) d'une première séquence d'ADN codant une protéine hétérologue ; (b) d'une deuxième séquence d'ADN codant un marqueur ; et (c) d'une troisième séquence d'ADN codant un facteur de virulence de *Salmonella* inactivé par mutation, ledit facteur de virulence étant codé par un gène *pag* ou *prg* qui a été inactivé par mutation autrement que par insertion d'un gène d' antibiorésistance.
